(19) 

Europäisches
Patentamt

European
Patent Office

Office européen
des brevets

(11) **EP 4 625 435 A1**

(12) **EUROPEAN PATENT APPLICATION**

published in accordance with Art. 153(4) EPC

(43) Date of publication:
   **01.10.2025 Bulletin 2025/40**

(21) Application number: **24791994.7**

(22) Date of filing: **16.04.2024**

(51) International Patent Classification (IPC):
   **G16H 40/67** (2018.01)

(52) Cooperative Patent Classification (CPC):
   **G16H 10/60; G16H 40/67; G16H 50/30**

(86) International application number:
   **PCT/CN2024/088027**

(87) International publication number:
   **WO 2024/217409 (24.10.2024 Gazette 2024/43)**

(84) Designated Contracting States:
   **AL AT BE BG CH CY CZ DE DK EE ES FI FR GB
   GR HR HU IE IS IT LI LT LU LV MC ME MK MT NL
   NO PL PT RO RS SE SI SK SM TR**
   Designated Extension States:
   **BA**
   Designated Validation States:
   **GE KH MA MD TN**

(30) Priority: **20.04.2023 CN 202310461060**

(71) Applicant: **Huawei Technologies Co., Ltd.
   Shenzhen, Guangdong 518129 (CN)**

(72) Inventors:
   • **ZHANG, Jie
     Shenzhen, Guangdong 518129 (CN)**

   • **XIE, Songlin
     Shenzhen, Guangdong 518129 (CN)**
   • **PAN, Jun
     Shenzhen, Guangdong 518129 (CN)**
   • **TIAN, Kan
     Shenzhen, Guangdong 518129 (CN)**
   • **ZHAO, Qifan
     Shenzhen, Guangdong 518129 (CN)**

(74) Representative: **Maiwald GmbH
   Engineering
   Elisenhof
   Elisenstrasse 3
   80335 München (DE)**

(54) **METHOD FOR DATA TRANSMISSION AND RELATED DEVICE**

(57)    This application discloses a data transmission method and a related device. In the method, a health detection device may determine a first data sampling interval based on a data amount of a first user health data set, a data transmission rate, and expected transmission waiting duration, sample the to-be-transmitted first user health data set at the first data sampling interval, to obtain first transmission data, and preferentially transmit the first transmission data in the first user health data set, so that a terminal device can output user health data and/or a related health indicator without waiting for a long time. Therefore, a user can view the user health data and/or the related health indicator as soon as possible, thereby effectively improving user experience.

| Health detection device | Terminal device |
| --- | --- |
| S301: Obtain a first user health data set | |
| S302: Sample the to-be-transmitted first user health data set at a first data sampling interval, to obtain first transmission data | |
| S303: First transmission data → | |
| | S304: Output the first transmission data |

FIG. 3

**EP 4 625 435 A1**

## Description

This application claims priority to Chinese Patent Application No. 202310461060.2, filed with the China National Intellectual Property Administration on April 20, 2023 and entitled "DATA TRANSMISSION METHOD AND RELATED DEVICE", which is incorporated herein by reference in its entirety.

## TECHNICAL FIELD

This application relates to the field of terminal technologies, and in particular, to a data transmission method and a related device.

## BACKGROUND

In recent years, prevalence of diabetes has increased year by year due to influence of many factors such as living standard, diet structure and sub-health lifestyle. For effective blood glucose control, technologies related to blood glucose monitoring are gradually evolving towards intelligence and informatization. Therefore, continuous glucose monitoring (CGM) has attracted wide attention from personnel in related fields.

A user may complete continuous glucose monitoring by using a health detection device and a terminal device. When blood glucose data detected by the health detection device is synchronously uploaded to the terminal device, the terminal device may synchronously manage the blood glucose data, and may output the blood glucose data and/or a related blood glucose indicator on the terminal device, to facilitate formulation of a diabetes therapy plan. However, if a blood glucose management function is not enabled for a long time, once the function is enabled, the blood glucose data and/or the related blood glucose indicator can be output only after a long time of waiting, resulting in poor user experience.

## SUMMARY

This application provides a data transmission method and a related device. A terminal device may output user health data and/or a related health indicator without waiting for a long time, so that a user can view the user health data and/or the related health indicator as soon as possible.

According to a first aspect, an embodiment of this application provides a data transmission method. The method may be applied to a health detection device, or may be applied to a chip in the health detection device, or may be applied to a logic module or software that can implement all or some functions of the health detection device. The method includes: obtaining a first user health data set that includes user health data detected at a plurality of detection time points, sampling the to-be-transmitted first user health data set at a first data sampling interval, to obtain first transmission data, and transmitting the first transmission data to a terminal device, where the first data sampling interval is determined based on a data amount of the first user health data set, a data transmission rate, and expected transmission waiting duration.

In embodiments of this application, the health detection device may preferentially transmit some data (that is, the first transmission data) in the first user health data set. The first transmission data is obtained by performing sampling at the first data sampling interval, and may represent an overall change trend of user health data. The expected transmission waiting duration is taken into account for the first data sampling interval. Therefore, the terminal device can output user health data and/or a related health indicator without waiting for a long time, so that a user can view the user health data and/or the related health indicator as soon as possible, thereby effectively improving user experience. In addition, the data transmission rate is further taken into account for obtaining the first data sampling interval of the first transmission data. Therefore, when a communication connection is unstable or the data transmission rate is low, the terminal device can also output the user health data and/or the related health indicator without waiting for a long time, thereby effectively improving user experience.

Optionally, the user health data includes one or more of blood glucose data, body composition data, blood pressure data, or blood lipid data. This is not limited.

Optionally, the health detection device may calculate the first data sampling interval by using the following formula:

$$S_1 = \left[ \frac{D_1}{v} \times \frac{1}{T_0} \right] + 1 \qquad (1)$$

$S_1$ is the first data sampling interval, $D_1$ is the data amount of the first user health data set, $v$ is the data transmission rate, $T_0$ is the expected transmission waiting duration, and [] is a rounding symbol.

Optionally, the expected transmission waiting duration $T_0$ is maximum duration that the user can accept to wait for data transmission.

With reference to the first aspect, in an optional implementation, detection time points of all pieces of user health data in the first user health data set are within a first time interval. It can be learned that the health detection device may preferentially transmit the user health data in the first time interval, so that the terminal device may preferentially output the user health data and/or the related health indicator in the first time interval.

With reference to the first aspect, in an optional implementation, the first time interval is a time interval of last 24 hours. In this implementation, importance of user health data within the last 24 hours is relatively high, and the health detection device may preferentially transmit the user health data within the last 24 hours, so that the terminal device may preferentially output the user health data and/or a related health indicator within the last 24 hours.

With reference to the first aspect, in an optional implementation, the method further includes: sampling a second user health data set at a second data sampling interval to obtain second transmission data, and transmitting the second transmission data to the terminal device. The second user health data set includes a plurality of pieces of user health data whose detection time points are outside the first time interval, and the second data sampling interval is determined based on a data amount of the second user health data set, the data transmission rate, and the expected transmission waiting duration.

With reference to the first aspect, in an optional implementation, detection time points of all pieces of user health data in the second user health data set are within a second time interval.

With reference to the first aspect, in an optional implementation, the second time interval is a time interval between last 48 hours and last 24 hours.

Optionally, the first user health data set and the second user health data set are obtained by dividing to-be-transmitted data based on detection time points. For example, the first time interval is the time interval of the last 24 hours, and the second time interval is the time interval between the last 24 hours and the last 48 hours. In this case, the to-be-transmitted data may include the first user health data set whose detection time points are within the last 24 hours, and the second user health data set whose detection time points are between the last 24 hours and the last 48 hours.

In this implementation, the health detection device may divide the to-be-transmitted data into a plurality of user health data sets based on the detection time points, and detection time points of user health data in the different user health data sets are within different time intervals. This helps the health detection device sequentially transmit the plurality of user health data sets based on an importance order of the user health data in all time intervals.

With reference to the first aspect, in an optional implementation, after transmitting the second transmission data to the terminal device, the method further includes: transmitting third transmission data to the terminal device, where the third transmission data is a part or all of the second user health data set except the second transmission data. It can be learned that the health detection device may transmit all data other than the second transmission data in the second user health data set at a time, or may transmit data other than the second transmission data in the second user health data set step by step.

With reference to the first aspect, in an optional implementation, after transmitting the first transmission data to the terminal device, the method further includes: transmitting fourth transmission data to the terminal device, where the fourth transmission data is a part or all of the first user health data set except the first transmission data. It can be learned that the health detection device may transmit all data other than the first transmission data in the first user health data set at a time, or may transmit data other than the first transmission data in the first user health data set step by step.

With reference to the first aspect, in an optional implementation, the first transmission data includes user health data detected last time. In this implementation, the first transmission data includes the user health data detected last time, so that the user can view, as soon as possible, the user health data that is detected last time and that is most concerned by the user. For example, when the blood glucose data is detected at an interval of one minute, the first transmission data includes information such as a blood glucose value in the last minute and a blood glucose change trend, so that the user can view the information such as the blood glucose value detected in the last minute and the blood glucose change trend as soon as possible.

With reference to the first aspect, in an optional implementation, the method further includes: receiving the expected transmission waiting duration from the terminal device.

According to a second aspect, an embodiment of this application provides another data transmission method. The method may be applied to a terminal device, or may be applied to a chip in the terminal device, or may be applied to a logic module or software that can implement all or some functions of the terminal device. The method includes: receiving first transmission data from a health detection device, and outputting the first transmission data, where the first transmission data is obtained by the health detection device by sampling a first user health data set at a first data sampling interval, the first user health data set includes user health data detected at a plurality of detection time points, and the first data sampling interval is determined based on a data amount of the first user health data set, a data transmission rate, and expected transmission waiting duration.

In embodiments of this application, the first transmission data received by the terminal device may represent an overall change trend of user health data. In addition, the first transmission data is received within the expected transmission waiting duration, and the terminal device can output the user health data and/or a related health indicator without waiting for a long time, so that a user can view the user health data and/or the related health indicator as soon as possible, thereby effectively improving user experience. In addition, the first data sampling interval for the received first transmission data is further related to the data transmission rate. Therefore, when a communication connection is unstable or the data transmission rate is low, the terminal device can also output the user health data and/or the related health indicator without waiting for a long time, thereby effectively improving user experience.

With reference to the second aspect, in an optional implementation, detection time points of all pieces of user health data in the first transmission data are within a first time interval. It can be learned that the terminal device may preferentially output the user health data and/or the related health indicator in the first time interval.

With reference to the second aspect, in an optional implementation, the first time interval is a time interval of last 24 hours. In this implementation, importance of user health data within the last 24 hours is relatively high, and the terminal device may preferentially output the user health data and/or a related health indicator within the last 24 hours.

With reference to the second aspect, in an optional implementation, the method further includes: receiving second transmission data from the health detection device, and outputting the second transmission data, where the second transmission data is obtained by the health detection device by sampling a second user health data set at a second data sampling interval, the second user health data set includes a plurality of pieces of user health data whose detection time points are outside the first time interval, and the second data sampling interval is determined based on a data amount of the second user health data set, the data transmission rate, and the expected transmission waiting duration.

With reference to the second aspect, in an optional implementation, detection time points of all pieces of user health data in the second transmission data are within a second time interval.

With reference to the second aspect, in an optional implementation, the second time interval is a time interval between last 48 hours and last 24 hours.

In this implementation, when importance of data in the first time interval is higher than importance of data in the second time interval, the terminal device may first receive the first transmission data in the first time interval, and then receive the second transmission data in the second time interval. The terminal device can output the user health data and/or the related health indicator that are/is in the first time interval and that have/has relatively high importance without waiting for a long time.

With reference to the second aspect, in an optional implementation, after receiving the second transmission data from the health detection device, the method further includes: receiving third transmission data from the health detection device, and outputting the third transmission data, where the third transmission data is a part or all of the second user health data set except the second transmission data. It can be learned that the terminal device may output data other than the second transmission data in the second user health data set at a time, or may output data other than the second transmission data in the second user health data set step by step.

With reference to the second aspect, in an optional implementation, after receiving the first transmission data from the health detection device, the method further includes: receiving fourth transmission data from the health detection device, and outputting the fourth transmission data, where the fourth transmission data is a part or all of the first user health data set except the first transmission data.

With reference to the second aspect, in an optional implementation, the first transmission data includes user health data detected last time.

With reference to the second aspect, in an optional implementation, the expected transmission waiting duration is sent to the health detection device.

According to a third aspect, an embodiment of this application further provides a health detection device, including a memory, one or more processors, a plurality of applications, and one or more programs, where the one or more programs are stored in the memory, and when the one or more processors execute the one or more programs, the health detection device is enabled to implement the method according to the first aspect.

According to a fourth aspect, an embodiment of this application further provides a terminal device, including a touchscreen, a memory, one or more processors, a plurality of applications, and one or more programs, where the one or more programs are stored in the memory, and when the one or more processors execute the one or more programs, the terminal device is enabled to implement the method according to the second aspect.

According to a fifth aspect, an embodiment of this application further provides a computer storage medium, including computer instructions. When the computer instructions are run on a health detection device, the health detection device is enabled to perform the method according to the first aspect, or when the computer instructions are run on a terminal device, the terminal device is enabled to perform the method according to the second aspect.

According to a sixth aspect, an embodiment of this application further provides a computer program product. When the computer program product runs on a computer, the computer is enabled to perform the method according to the first aspect or the method according to the second aspect.

**BRIEF DESCRIPTION OF DRAWINGS**

To describe technical solutions in embodiments of this application more clearly, the following describes the accompanying drawings used in embodiments of this application.

FIG. 1 is a diagram of an architecture of a data processing system according to an embodiment of this application;
FIG. 2(a) and FIG. 2(b) are a diagram of a user interface according to an embodiment of this application;
FIG. 3 is a schematic flowchart of a data transmission method according to an embodiment of this application;
FIG. 4A to FIG. 4C are diagrams of sampling processing according to an embodiment of this application;
FIG. 5A to FIG. 5E are diagrams of another user interface according to an embodiment of this application;
FIG. 6(a) to FIG. 6(c) are a diagram of still another user interface according to an embodiment of this application;
FIG. 7 is a schematic flowchart of another data transmission method according to an embodiment of this application;
FIG. 8A-1 to FIG. 8B-2 are diagrams of yet another user interface according to an embodiment of this application;
FIG. 9 is a schematic flowchart of still another data transmission method according to an embodiment of this application;
FIG. 10A-1 to FIG. 10D-2 are diagrams of still yet another user interface according to an embodiment of this application;
FIG. 11 is a diagram of yet another data transmission method according to an embodiment of this application;
FIG. 12 is a diagram of a structure of an electronic device according to an embodiment of this application; and
FIG. 13 is a diagram of a software architecture according to an embodiment of this application.

**DESCRIPTION OF EMBODIMENTS**

The following clearly and completely describes the technical solutions according to embodiments of this application in detail with reference to the accompanying drawings. In descriptions of embodiments of this application, unless otherwise specified, "/" indicates "or". For example, A/B may indicate A or B. The term "and/or" in this specification merely describes an association relationship between associated objects, and indicates that three relationships may exist. For example, A and/or B may indicate the following three cases: Only A exists, both A and B exist, and only B exists. In addition, in the descriptions of embodiments of this application, "a plurality of" means two or more.

The following terms "first" and "second" are merely intended for a purpose of description, and shall not be understood as an indication or implication of relative importance or implicit indication of a quantity of indicated technical features. Therefore, a feature limited by "first" and "second" may explicitly or implicitly include one or more features. In descriptions of embodiments of this application, unless otherwise specified, "a plurality of" means two or more.

In embodiments of this application, "equal to" may be used together with "greater than", which is applicable to a technical solution used in a case of "greater than"; or may be used together with "less than", which is applicable to a technical solution used in a case of "less than". It should be noted that, when used together with "greater than", "equal to" is not used together with "less than"; and when used together with "less than", "equal to" is not used together with "greater than".

A term "user interface (user interface, UI)" in the following embodiments of this application is a medium interface for interaction and information exchange between an application or an operating system and a user, and implements conversion between an internal form of information and a form that can be accepted by the user. The user interface is source code written in a specific computer language such as Java or an extensible markup language (extensible markup language, XML). Interface source code is parsed and rendered on an electronic device, and is finally presented as content that can be identified by the user. A frequently-used representation form of the user interface is a graphical user interface (graphical user interface, GUI), and is a user interface that is output in a graphical manner and that is related to a computer operation. The user interface may be a visual interface element like a text, an icon, a button, a menu, a tab, a text box, a dialog box, a status bar, a navigation bar, or a widget that is output on an output display of the electronic device.

In embodiments of this application, the terms "system" and "network", and "collect" and "detect" may be used interchangeably, and meanings of the terms may be understood by persons skilled in the art.

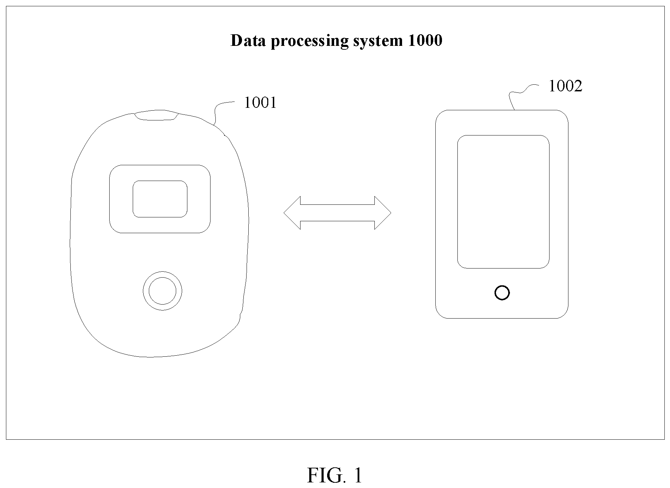

A data transmission method in embodiments of this application may be applied to a data processing system. As shown in FIG. 1, the example data processing system 1000 may include a health detection device 1001 and a terminal device 1002. The health detection device 1001 may be configured to detect or collect user health data (such as body composition data, blood pressure data, blood glucose data, and blood lipid data). The health detection device 1001 may include but is not limited to one or more of a body fat scale, a blood pressure meter, a blood glucose meter, a continuous glucose monitoring system (CGMS), or a blood lipid meter. The terminal device 1002 may be configured to manage user health data collected by various health detection devices 1001, and collect statistics on and analyze the managed user health data, so that a user can view the user health data and/or related health indicators. The terminal device 1002 may include but is not limited to one or more of a wearable electronic device (such as smart glasses or a smart watch), a scanner, a smartphone, a tablet computer, or a notebook computer. In some embodiments, the health detection device 1001 and the terminal device 1002 may be deployed in a same device, or may be deployed in different devices. This is not limited. It may be understood that, in actual application, the data processing system 1000 may include more or fewer devices. This is not limited herein.

Optionally, the health detection device 1001 and the terminal device 1002 may be connected to a local area network in a wired or wireless fidelity (wireless fidelity, Wi-Fi) connection manner, or may communicate with each other through a mobile network or the Internet, or may establish a communication connection in a near field communication (near field communication, NFC) or Bluetooth (Bluetooth) manner to implement data communication.

For example, the health detection device 1001 and the terminal device 1002 may be in a same local area network, and a Wi-Fi link is established between the health detection device 1001 and the terminal device 1002 according to a Wi-Fi protocol, to implement communication between the devices. Specifically, the health detection device 1001 and the terminal device 1002 establish a peer to peer (peer to peer, P2P) connection, or access a same router.

Optionally, a Bluetooth link may alternatively be established between the health detection device 1001 and the terminal device 1002 according to a Bluetooth protocol, to implement communication between the devices based on the Bluetooth link. Alternatively, the health detection device 1001 and the terminal device 1002 may be interconnected through a cellular network, or the health detection device 1001 and the terminal device 1002 may be interconnected through a transfer device (for example, a USB data line or a dock device), to implement communication.

Optionally, the terminal device may manage the user health data by using a health management function. The health management function may be a function of an application, or may be an independent application (for example, a health management application). In embodiments of this application, an example in which the health management function is an independent application is used for description.

In embodiments of this application, a blood glucose management function in the health management function is used as an example for description. The data processing system may manage blood glucose data based on a continuous glucose monitoring (continuous glucose monitoring, CGM) technology. The CGM technology is a new blood glucose detection technology. A health detection device is embedded in a user body, to continuously detect blood glucose data, and transmit the detected blood glucose data to a terminal device for data synchronization, so that the terminal device manages the blood glucose data, and a user can obtain the blood glucose data and/or a related blood glucose indicator.

The following describes an example user interface of a blood glucose management function on the terminal device 1002.

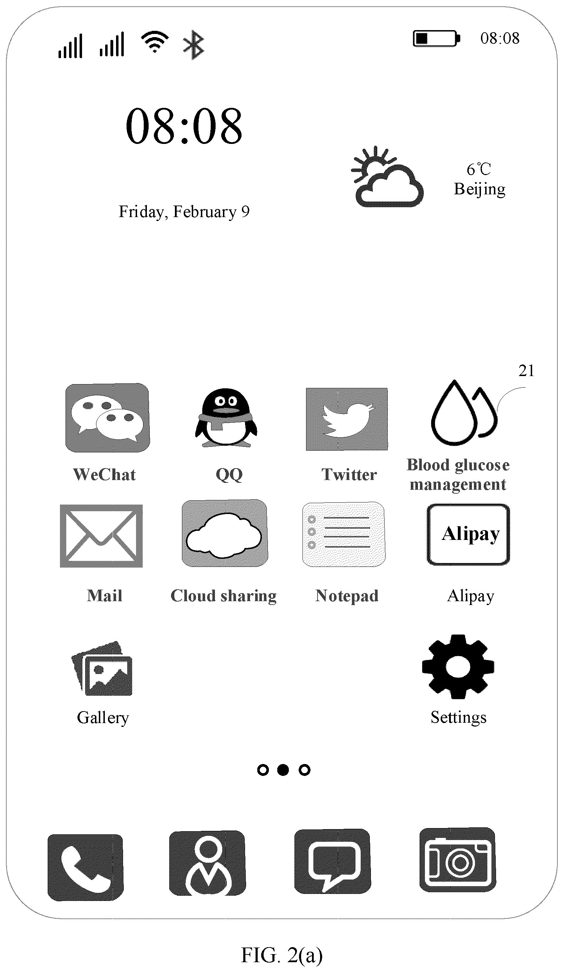

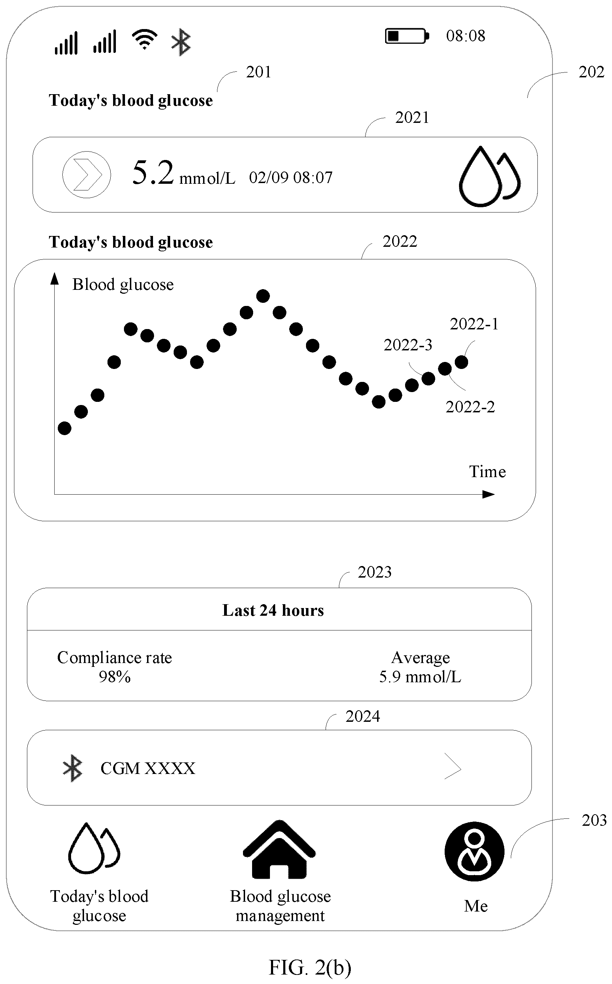

FIG. 2(a) and FIG. 2(b) are a diagram of an application interface according to an embodiment of this application. As shown in FIG. 2(a), an application icon (for example, a blood glucose management icon 21 shown in FIG. 2(a)) and another application icon corresponding to a blood glucose management application may be displayed on a screen interface of the terminal device. The user may tap the blood glucose management icon 21 to start the blood glucose management application. As shown in FIG. 2(b), in response to the operation of tapping the blood glucose management icon 21 by the user, the terminal device may display a main interface of the blood glucose management application. The main interface may include a function name 201, a card list 202, and a navigation bar 203.

The function name 201 may indicate a currently enabled function, for example, a "today's blood glucose" function shown in the figure. The card list 202 may include cards corresponding to various blood glucose data provided by the blood glucose management application, for example, a current blood glucose card 2021, a today's blood glucose card 2022, a blood glucose statistics card 2023, and a device connection card 2024 shown in the figure. The current blood glucose card 2021 may be used to display blood glucose data detected at a current detection time point, the today's blood glucose card 2022 may be used to display blood glucose data detected in the last 24 hours, the blood glucose statistics card 2023 may be used to display a blood glucose indicator (for example, a blood glucose compliance rate, an average blood glucose value, and the like) in the last 24 hours, and the device connection card 2024 may be used to display a data transmission status of the health detection device 1001. The navigation bar 203 may include various function menus as shown in FIG. 2(b), for example, a "today's blood glucose" function used to view various blood glucose data, a "blood glucose management" function used to view blood glucose indicators related to the various blood glucose data, and a "me" function used to perform personal account management.

However, if the terminal device does not enable the health management function for a long time (for example, the blood glucose management function is not enabled for a long time), excessive user health data collected by the health detection device is accumulated, and excessive data is to be synchronized. Once the function is enabled, it takes a long time to output user health data and/or a related health indicator, resulting in poor user experience. In view of this, embodiments of this application provide a data transmission method. In the method, a health detection device may preferentially transmit some data (that is, first transmission data) in a first user health data set. The first transmission data is obtained by performing sampling at a first data sampling interval, and may represent an overall change trend of user health data. Expected transmission waiting duration is taken into account for the first data sampling interval. Therefore, an output terminal device can output the user health data and/or a related health indicator without waiting for a long time, so that the user can view the user health data and/or the related health indicator as soon as possible, thereby effectively improving user experience.

The following describes a data transmission method according to an embodiment of this application with reference to the accompanying drawings.

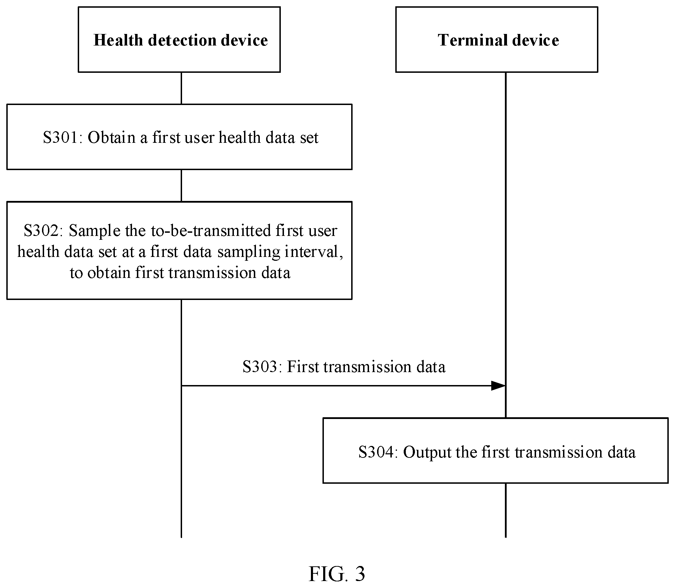

FIG. 3 is a schematic flowchart of a data transmission method according to an embodiment of this application. The data transmission method is described with reference to data exchange between a health detection device and a terminal device. As shown in FIG. 3, the method includes but is not limited to S301 to S304.

S301: The health detection device obtains a first user health data set, where the first user health data set includes user health data detected at a plurality of detection time points.

Optionally, the user health data includes one or more of blood glucose data, body composition data, blood pressure data, or blood lipid data. This is not limited. For example, the user health data is blood glucose data. The blood glucose data may include one or more of a blood glucose value, a blood glucose change trend, blood glucose alarm information, or blood glucose warning information.

It should be noted that the user health data has a corresponding detection time point (which may also be referred to as a "collection time point"). For example, as shown in FIG. 2(b), for blood glucose data "5.2 millimoles/liter (mmol/L)", a corresponding detection time point may be "08:07 on February 9".

Optionally, an interval between adjacent detection time points may be a fixed value, for example, the fixed value is one minute, that is, the health detection device collects user health data at an interval of one minute, and refreshes the user health data included in the first user health data set. It should be noted that the fixed value is not limited in this application.

It may be understood that, when detecting the user health data at the detection time point, the health detection device may store the user health data. Because a storage capability of the health detection device is limited, the first user health data set may have a maximum value. For example, the first user health data set includes at most all user health data detected in the last 16 days.

S302: The health detection device samples the to-be-transmitted first user health data set at a first data sampling interval, to obtain first transmission data, where the first data sampling interval is determined based on a data amount of the first user health data set, a data transmission rate, and expected transmission waiting duration.

The first transmission data is user health data in the first user health data set that is preferentially transmitted.

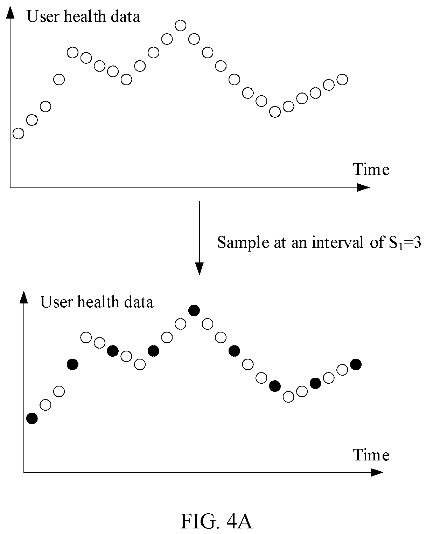

For example, the first user health data set includes user health data detected at 25 detection time points, and the first data sampling interval is S1=3. As shown in FIG. 4A, a white circle represents user health data in the first user health data set, and a black circle represents user health data in the first transmission data. After the first user health data set is sampled at the first data sampling interval S1=3, obtained first transmission data may include user health data detected at nine detection time points.

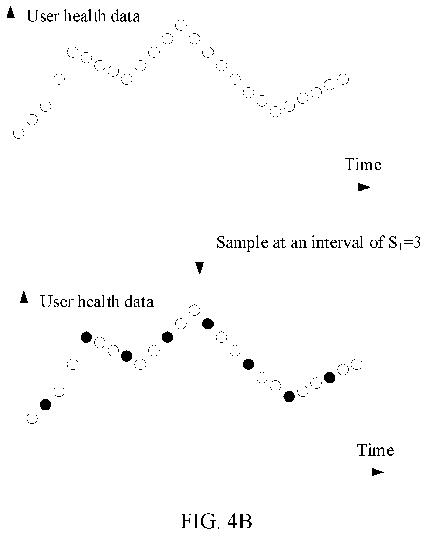

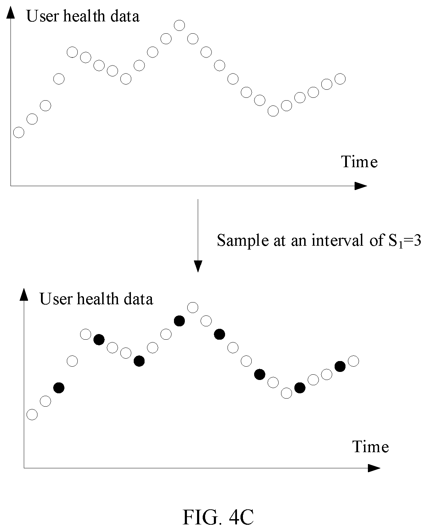

Optionally, a 1st sampling point in a sampling process may be an $i^{th}$ piece of user health data in the first user health data set, where i is a positive integer less than or equal to S1. S1=3 is used as an example. As shown in FIG. 4A, the 1st sampling point may be a 1st piece of user health data in the first user health data set. As shown in FIG. 4B, the 1st sampling point may be a 2nd piece of user health data in the first user health data set. As shown in FIG. 4C, the 1st sampling point may be a 3rd piece of user health data in the first user health data set. This is not limited.

Optionally, the first transmission data includes user health data detected last time. It can be learned that, in this implementation, the first transmission data includes the user health data detected last time, so that a user can view, as soon as possible, the user health data that is detected last time and that is most concerned by the user. For example, when the blood glucose data is detected at an interval of one minute, the first transmission data includes information such as a blood glucose value in the last minute and a blood glucose change trend, so that the user can view the information such as the blood glucose value detected in the last minute and the blood glucose change trend as soon as possible.

Optionally, the health detection device may calculate the first data sampling interval by using the following formula:

$$S_1 = \left[ \frac{D_1}{v} \times \frac{1}{T_0} \right] + 1 \qquad (1)$$

S1 is the first data sampling interval, D1 is the data amount of the first user health data set, v is the data transmission rate, T0 is the expected transmission waiting duration, and [] is a rounding symbol.

Optionally, the expected transmission waiting duration T0 is maximum duration that the user can accept to wait for data transmission. The expected transmission waiting duration may be set by a system by default or defined by the user. For example, when the expected transmission waiting duration is defined by the user, the user may set the expected transmission waiting duration through the terminal device and/or the health detection device. Optionally, when the transmission waiting duration is defined by the user through the terminal device, the health detection device may obtain the transmission waiting duration from the terminal device. It should be noted that a value of the expected transmission waiting duration is not limited in embodiments of this application. For example, the expected transmission waiting duration may be 5 seconds (s), 10 seconds (s), or the like.

For example, the data amount of the first user health data set is 50 kilobytes (kb), the data transmission rate is 2 kilobytes/second (kb/s), and the expected transmission waiting duration is 10s. That the first data sampling interval S1 is equal to 3 may be obtained by using Formula (1).

Optionally, when the data transmission rate v and the expected transmission waiting duration T0 remain unchanged, the first data sampling interval may vary with the data amount of the first user health data set. For example, when the data transmission rate is still 2 kb/s, the expected transmission waiting duration is still 10s, and the data amount of the first user health data set is 100 kilobytes (kb), the first data sampling interval is S1=6. It can be learned that the first data sampling interval is more flexible, and therefore, an interval at which the user health data in the first transmission data is output in the terminal device is also more flexible. In an optional implementation, when detecting a data transmission trigger operation, the health detection device performs step S302. For example, when the health detection device detects that a communication connection to the terminal device is established, and determines that the data transmission trigger operation is detected, the health detection device performs step S302. Specifically, the terminal device may start a "health management application", and select a found health detection device for connection. When the connection succeeds, step S302 may be performed. For another example, when the health detection device receives a data upload instruction from the terminal device, and determines that the data transmission trigger operation is detected, the health detection device may perform step S302. Optionally, the data upload instruction may carry a parameter required for data transmission, for example, the expected transmission waiting duration and/or the data transmission rate. It should be noted that the data upload instruction may be triggered by the user, or may be periodically triggered by the terminal device. This is not limited.

S303: The health detection device transmits the first transmission data to the terminal device. Correspondingly, the terminal device receives the first transmission data from the health detection device.

Optionally, the health detection device completes transmission of the first transmission data in the first user health data set within the expected transmission waiting duration.

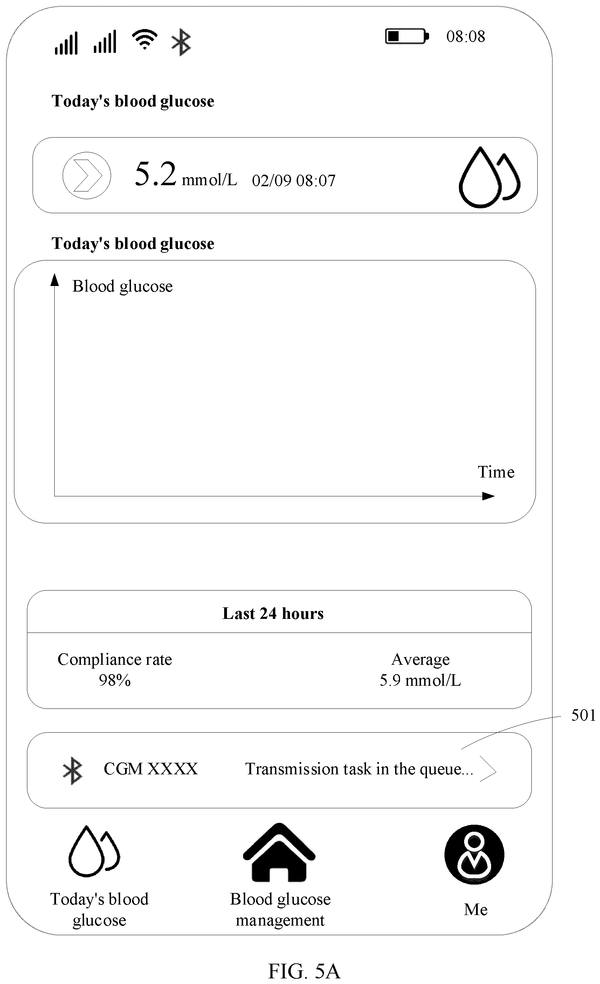

In some embodiments, as shown in one or more examples in FIG. 5A to FIG. 5D, the terminal device may output prompt information in a data transmission process. The prompt information may indicate a data transmission status of the first user health data set.

As shown in FIG. 5A, the prompt information 501 may indicate that the transmission status of the first user health data set is a first transmission state. The first transmission state may indicate that the first user health data set is in a transmission task queue and waiting to be uploaded by the health detection device. The prompt information 501 may be text information "Transmission task in the queue...", and is not limited thereto. The prompt information 501 may alternatively be information in another form, for example, a picture or an animation.

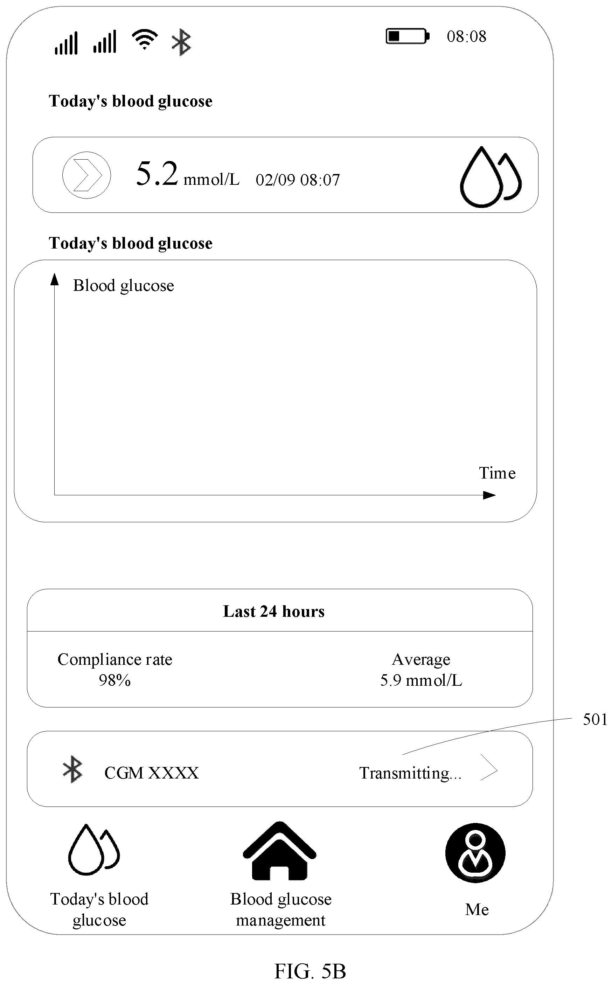

As shown in FIG. 5B, the prompt information 501 may indicate that the transmission status of the first user health data set is a second transmission state. The second transmission state may indicate that the first user health data set is being transmitted. The prompt information 501 may be text information "Transmitting...", and is not limited thereto. The prompt information 501 may alternatively be information in another form, for example, a picture or an animation.

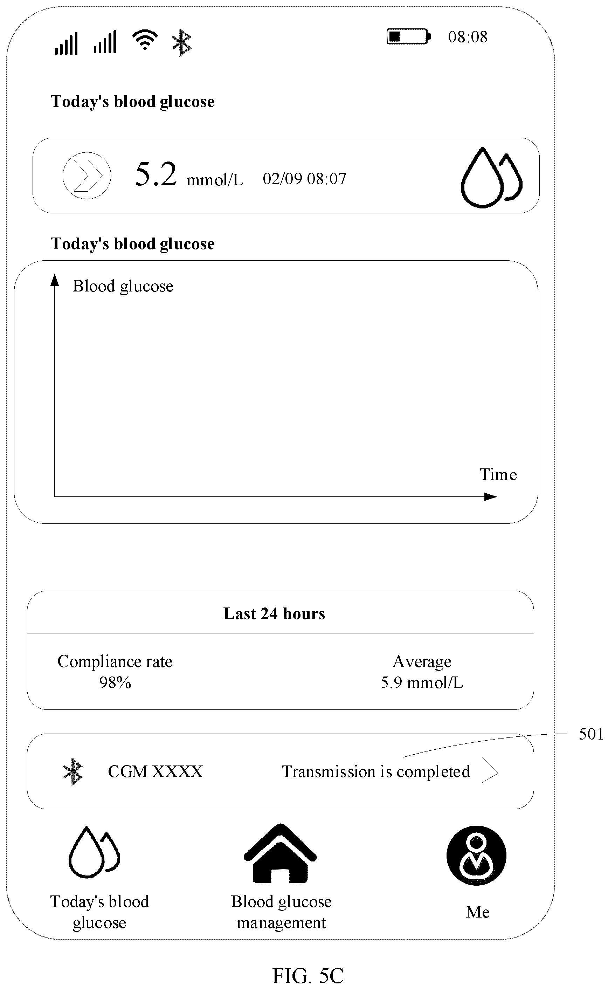

As shown in FIG. 5C, the prompt information 501 may indicate that the transmission status of the first user health data set is a third transmission state. The third transmission state may indicate that transmission of the first user health data set is completed. The prompt information 501 may be text information "Transmission is completed", and is not limited thereto. The prompt information 501 may alternatively be information in another form, for example, a picture or an animation.

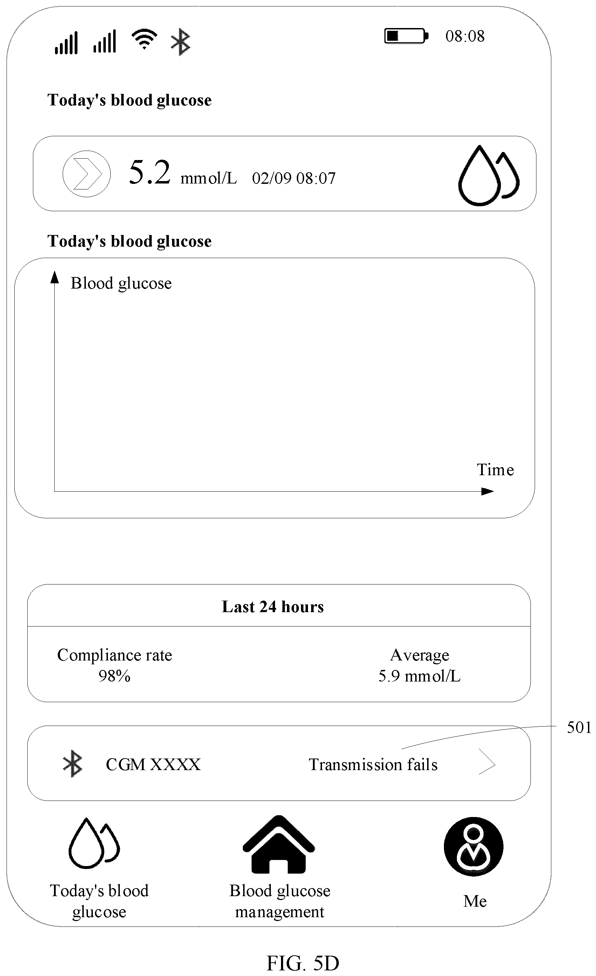

As shown in FIG. 5D, the prompt information 501 may indicate that the transmission status of the first user health data set is a fourth transmission state. The fourth transmission state may indicate that transmission of the first user health data set fails. The prompt information 501 may be text information "Transmission fails", and is not limited thereto. The prompt information 501 may alternatively be information in another form, for example, a picture or an animation.

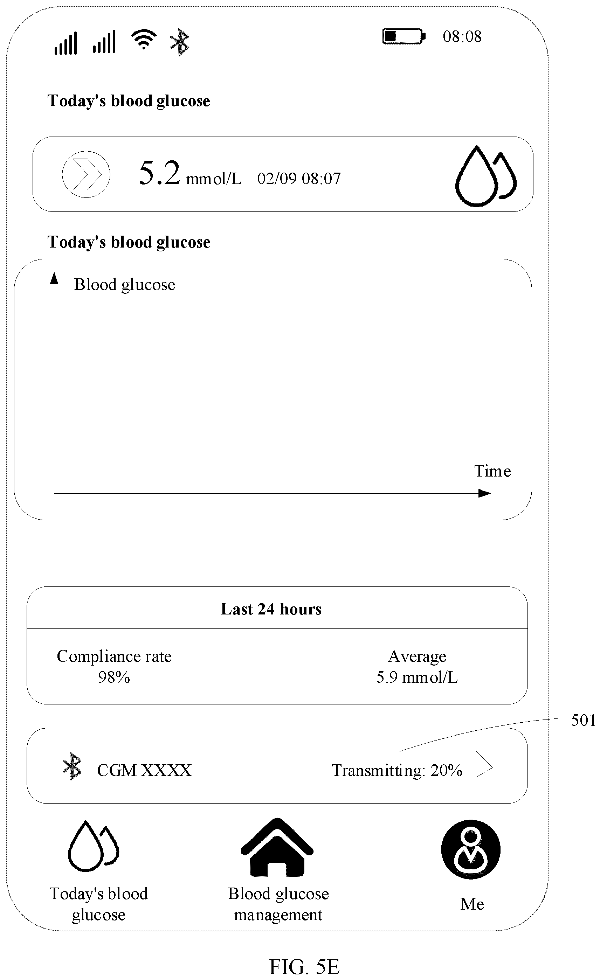

In some embodiments, the prompt information may further indicate a transmission progress. In other words, the second transmission state mentioned in the foregoing embodiment may be further refined as the transmission progress. For example, as shown in FIG. 5E, the prompt information may be text information "Transmitting: 20%". In this way, the user can learn the transmission progress of the first user health data set, which is more convenient, providing better user experience.

It should be noted that the foregoing prompt information may be located at any position on a screen interface. This is not limited in this application.

S304: The terminal device outputs the first transmission data.

In an optional implementation, when transmission of the first transmission data in the first user health data set is completed, the terminal device may sequentially output, on a screen of the terminal device based on a sequence of the detection time points of all pieces of user health data, all the pieces of the user health data in the first transmission data. It should be noted that the user health data output in embodiments of this application may be text information, image information, or animation information that is output through a screen, or may be voice information that is output through a speaker. This is not limited.

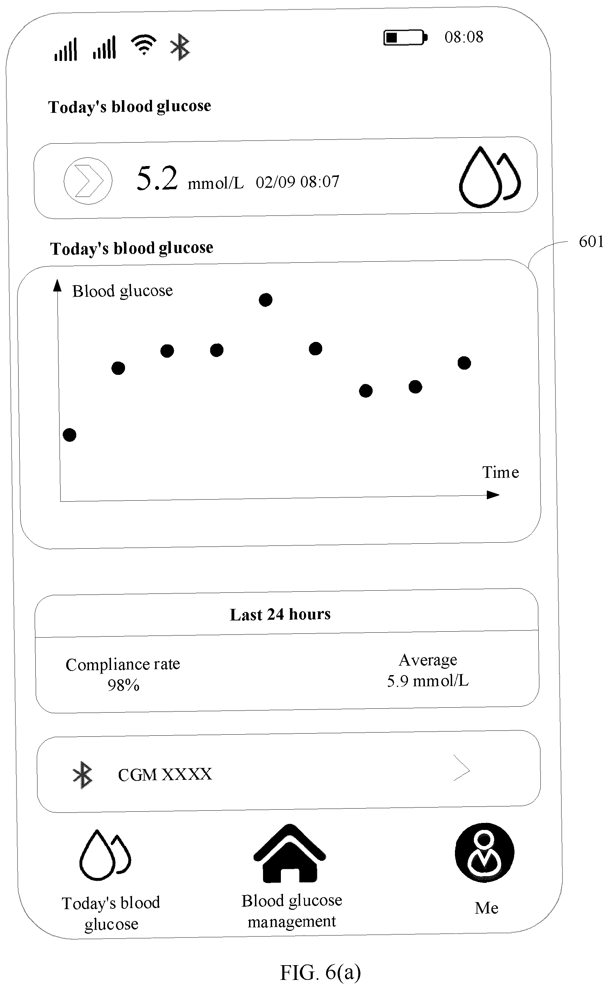

For example, the user health data is blood glucose data, and the user health data is image information output through a screen. As shown in FIG. 6(a), with reference to the example in FIG. 4A, blood glucose data detected at nine detection time points (that is, the first transmission data that is preferentially transmitted) may be output in a region 601.

Optionally, the terminal device may further perform statistical analysis on each piece of user health data in the first transmission data, to obtain a related health indicator, and output the related health indicator. For example, when the user health data is blood glucose data, the related health indicator includes but is not limited to one or more of an average blood glucose, a blood glucose variation coefficient, a blood glucose standard deviation, a blood glucose management indicator, a hypoglycemia index, a hyperglycemia index, an area under a curve, or a valid data ratio. It should be noted that related health data output in embodiments of this application may be text information, image information, or animation information that is output through a screen, or may be voice information that is output through a speaker. This is not limited.

It should be noted that, optionally, the foregoing related health indicator may alternatively be obtained by the health detection device through statistical analysis. In this case, the health detection device may transmit the related health indicator to the terminal device, so that the terminal device outputs the related health indicator.

Optionally, after step S304, the method may further include: The health detection device transmits fourth transmission data to the terminal device, and when the terminal device receives the fourth transmission data from the health detection device, the terminal device may output the fourth transmission data. The fourth transmission data is a part or all of the first user health data set except the first transmission data.

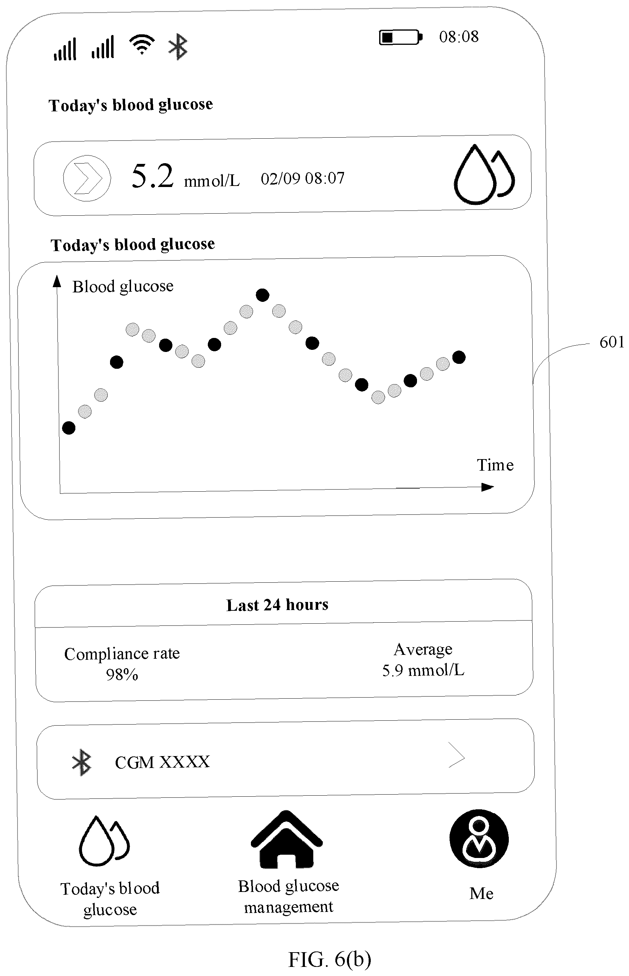

In some embodiments, the fourth transmission data is all of data other than the first transmission data in the first user health data set. For example, the first user health data set includes blood glucose data detected at 25 detection time points, and the first transmission data includes blood glucose data detected at nine detection time points. The fourth transmission data may be the remaining 16 pieces of blood glucose data. As shown in FIG. 6(b), a gray circle represents blood glucose data in the fourth transmission data. When receiving the fourth transmission data from the health detection device, the terminal device may output, in the region 601, the remaining blood glucose data (that is, the fourth transmission data) detected at 16 detection time points.

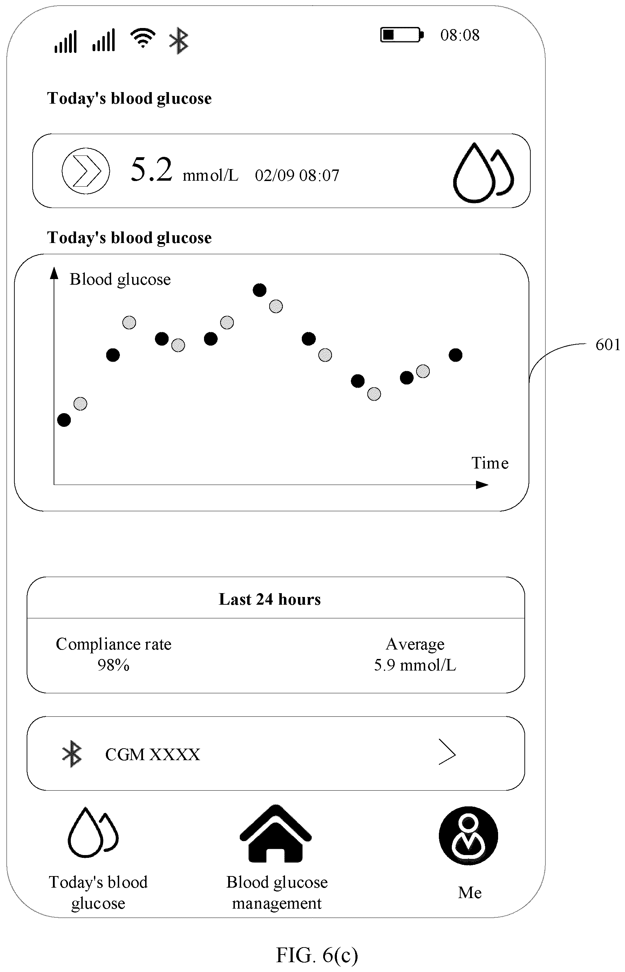

In some embodiments, the fourth transmission data is a part of data other than the first transmission data in the first user health data set. For example, the first user health data set includes blood glucose data detected at 25 detection time points, and the first transmission data includes blood glucose data detected at nine detection time points. The fourth transmission data may be eight pieces of blood glucose data in the remaining 16 pieces of blood glucose data. As shown in FIG. 6(c), a gray circle represents blood glucose data in the fourth transmission data. When receiving the fourth transmission data from the health detection device, the terminal device may output, in the region 601, the blood glucose data (that is, the fourth transmission data) detected at eight detection time points.

It should be noted that transmission duration for transmitting the fourth transmission data by the health detection device may be determined based on a data amount of the fourth transmission data and the data transmission rate. For example, when the data amount of the fourth transmission data is 40 kilobytes (kb) and the data transmission rate is 2 kilobytes/second (kb/s), the transmission duration may be obtained as follows: t=40/2=20s.

It should be noted that a step of transmitting, by the health detection device, the fourth transmission data to the terminal device may be triggered when a data transmission instruction from the terminal device is detected, or may be automatically triggered when the transmission of the first transmission data is completed. This is not limited.

It may be understood that, in a case in which the fourth transmission data is a part of data other than the first transmission data in the first user health data set, the health detection device may transmit remaining user health data that is not transmitted yet in the first user health data set again. Details are not described again.

In embodiments of this application, the health detection device may preferentially transmit some data (that is, the first transmission data) in the first user health data set. The first transmission data is obtained by performing sampling at the first data sampling interval, and may represent an overall change trend of user health data. The expected transmission waiting duration is taken into account for the first data sampling interval. Therefore, the output terminal device may output the user health data and/or the related health indicator without waiting for a long time, so that the user can view the user health data and/or the related health indicator as soon as possible, thereby effectively improving user experience. In addition, the data transmission rate is further taken into account for obtaining the first data sampling interval of the first transmission data. Therefore, when the communication connection is unstable or the data transmission rate is low, the terminal device can also output the user health data and/or the related health indicator without waiting for a long time, thereby effectively improving user experience.

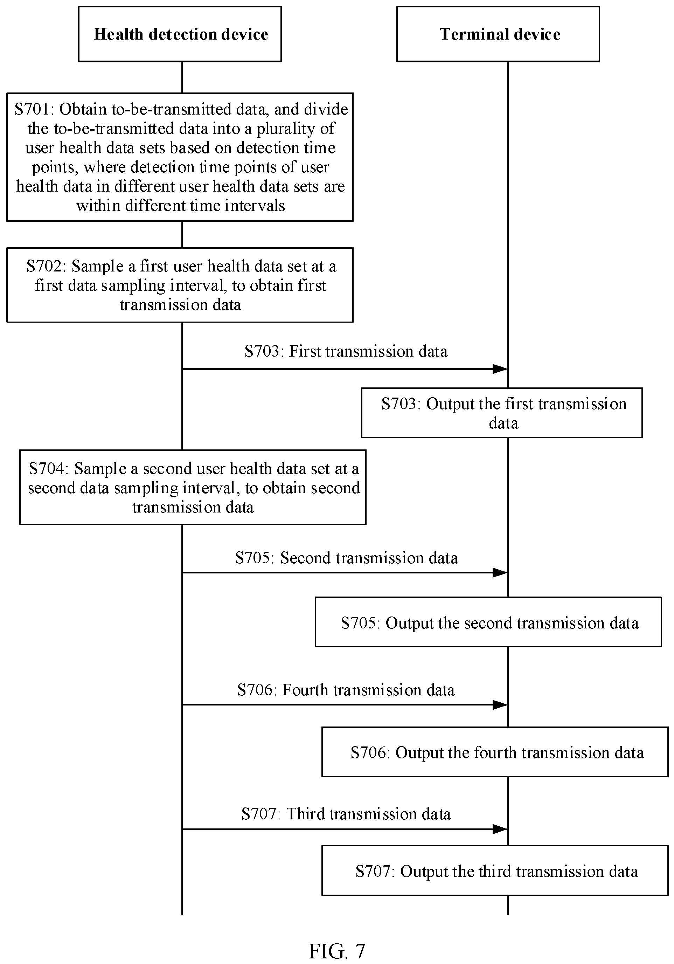

FIG. 7 is a schematic flowchart of another data transmission method. In comparison with the data transmission method in FIG. 3, in the data transmission method in FIG. 7, to-be-transmitted data may be divided into a plurality of user health data sets based on detection time points, where detection time points of user health data in the different user health data sets are within different time intervals. In this case, the health detection device may sequentially transmit the plurality of user health data sets based on an importance order of the user health data in all the time intervals. As shown in FIG. 7, the method includes but is not limited to S701 to S707.

S701: The health detection device obtains to-be-transmitted data, and divides the to-be-transmitted data into a plurality of user health data sets based on detection time points, where detection time points of user health data in the different user health data sets are within different time intervals.

For example, the health detection device divides the to-be-transmitted data into a first user health data set and a second user health data set based on the detection time points. Detection time points of all pieces of user health data in the first user health data set are within a first time interval, and detection time points of all pieces of user health data in the second user health data set are outside the first time interval. In other words, the first user health data set includes a plurality of pieces of user health data whose detection time points are within the first time interval, and the second user health data set includes a plurality of pieces of user health data whose detection time points are within the second time interval. The second time interval is any time interval other than the first time interval. For example, the first time interval is a time interval of last 24 hours, and the second time interval is a time interval outside the last 24 hours. For another example, the first time interval is a time interval of last 24 hours, and the second time interval is a time interval between last 48 hours and last 24 hours.

For another example, the health detection device divides the to-be-transmitted data into a first user health data set, a second user health data set, and a third user health data set based on the detection time points. The first user health data set includes a plurality of pieces of user health data whose detection time points are within a first time interval, the second user health data set includes a plurality of pieces of user health data whose detection time points are within a second time interval, and the third user health data set includes a plurality of pieces of user health data whose detection time points are within a third time interval, where the first time interval, the second time interval, and the third time interval are different time intervals. For example, the first time interval is a time interval of last 24 hours, the second time interval is a time interval between last 48 hours and last 24 hours, and the third time interval is a time interval between the last 48 hours and last 72 hours.

It should be noted that the foregoing plurality of user health data sets are merely used as examples for description. In another implementation, the to-be-transmitted data may be further divided into more user health data sets. Details are not described again.

It should be noted that for related descriptions of the to-be-transmitted data, refer to the related embodiment of step S301. Details are not described again.

It should be noted that the foregoing time interval is merely used as an example for description. In another implementation, the time interval may alternatively be a time interval of a natural day. For example, the first time interval is a time interval from 00:00 to 23:59 on February 9, the second time interval is a time interval from 00:00 to 23:59 on February 8, and the third time interval is a time interval from 00:00 to 23:59 on February 7. This is not limited.

It should be noted that, for ease of description, subsequent steps are described by using an example in which the to-be-transmitted data is divided into the first user health data set and the second user health data set, and importance of the first user health data set is higher than importance of the second user health data set.

S702: The health detection device samples the first user health data set at a first data sampling interval, to obtain first transmission data, where the first data sampling interval is determined based on a data amount of the first user health data set, a data transmission rate, and expected transmission waiting duration.

S703: The health detection device transmits the first transmission data to the terminal device, and when the terminal device receives the first transmission data from the health detection device, the terminal device outputs the first transmission data.

It should be noted that for related descriptions of steps S702 and S703, refer to steps S301 to S304. Details are not described again.

S704: The health detection device samples the second user health data set at a second data sampling interval, to obtain second transmission data, where the second data sampling interval is determined based on a data amount of the second user health data set, the data transmission rate, and the expected transmission waiting duration.

The second transmission data is user health data in the second user health data set that is preferentially transmitted.

Optionally, the health detection device may calculate the second data sampling interval by using the following formula:

$$S_2 = \left[ \frac{D_2}{v} \times \frac{1}{T_0} \right] + 1 \quad (2)$$

S2 is the second data sampling interval, D2 is the data amount of the second user health data set, v is the data transmission rate, T0 is the expected transmission waiting duration, and [] is a rounding symbol.

It should be noted that for related descriptions, refer to step S302. Details are not described again.

S705: The health detection device transmits the second transmission data to the terminal device, and when the terminal device receives the second transmission data from the health detection device, the terminal device outputs the second transmission data.

It should be noted that for related descriptions, refer to step S303 and step S304. Details are not described again.

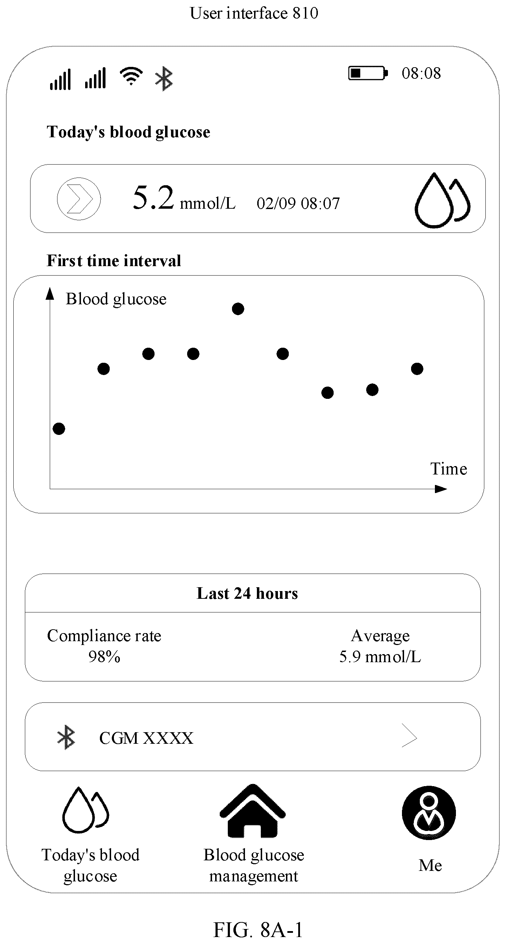

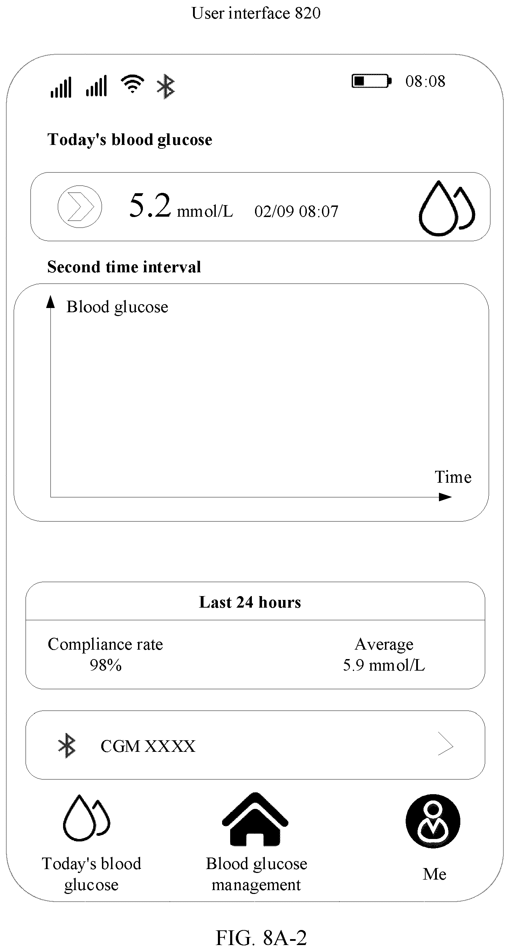

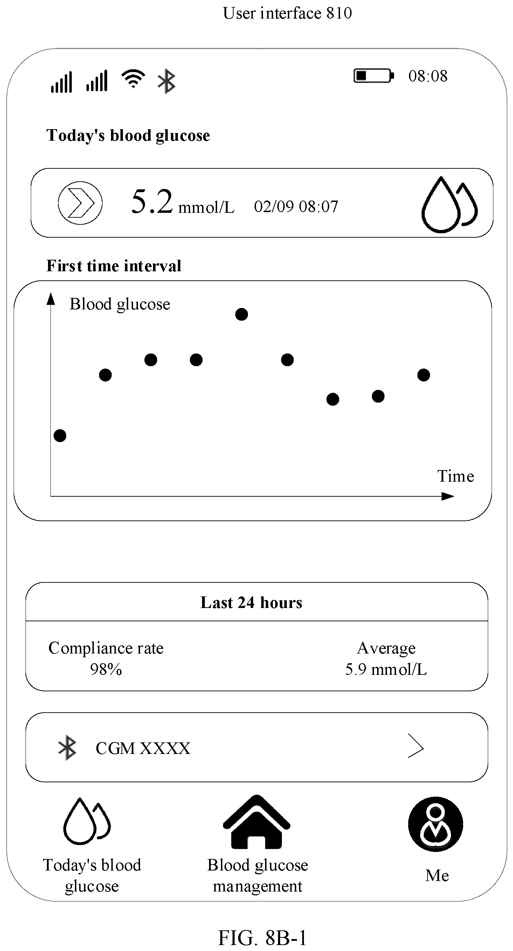

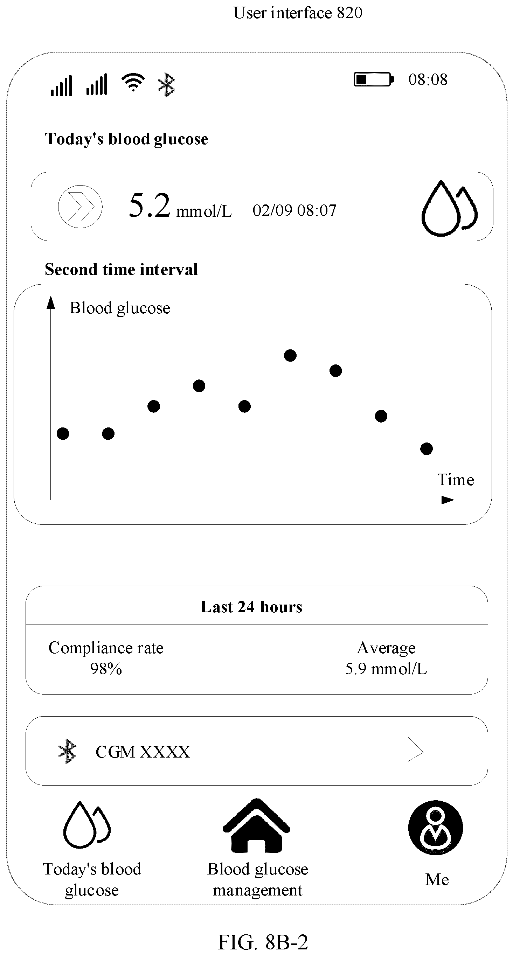

The following describes the embodiment in FIG. 7 with reference to the user interfaces in FIG. 8A-1 to FIG. 8B-2.

As shown in FIG. 8B-1 and FIG. 8B-2, when transmission of the first transmission data in the first user health data set is completed, and transmission of the second transmission data in the second user health data set is not completed (that is, transmission of preferentially transmitted data in the first time interval is completed, and transmission of preferentially transmitted data in the second time interval is not completed), the terminal device may sequentially output, in a user interface 810, nine pieces of blood glucose data whose detection time points are within the first time interval (that is, the preferentially transmitted data in the first time interval). In addition, when the terminal device detects a user operation (for example, a right sliding operation or a left sliding operation) on the user interface 810, and the terminal device switches from the user interface 810 to an output user interface 820, the user interface 820 has not output the blood glucose data whose detection time point is within the second time interval.

As shown in FIG. 8B-1 and FIG. 8B-2, when transmission of the first transmission data in the first user health data set is completed, and transmission of the second transmission data in the second user health data set is completed (that is, transmission of preferentially transmitted data in the first time interval is completed, and transmission of preferentially transmitted data in the second time interval is also completed), nine pieces of blood glucose data whose detection time points are within the first time interval (that is, preferentially transmitted data in the first time interval) may be sequentially output in a user interface 810, and in addition, blood glucose data whose detection time point is within the second time interval (that is, preferentially transmitted data in the second time interval) may be sequentially output in a user interface 820.

Optionally, after step S705, the method may further include step S706: The health detection device transmits fourth transmission data to the terminal device, and when the terminal device receives the fourth transmission data from the health detection device, the terminal device may output the fourth transmission data. The fourth transmission data is a part or all of the first user health data set except the first transmission data. For related descriptions, refer to the related embodiment in FIG. 3. Details are not described again.

Optionally, after step S705, the method may further include step S707: The health detection device transmits third transmission data to the terminal device, and when the terminal device receives the third transmission data from the health detection device, the terminal device may output the third transmission data. The third transmission data is a part or all of the second user health data set except the second transmission data. For related descriptions, refer to the related embodiment in FIG. 3. Details are not described again.

It should be noted that an execution sequence of step S706 and step S707 is not limited in this application. For example, step S706 may be performed first, and then step S707 is performed. For another example, step S707 may be performed first, and then step S706 is performed. For still another example, step S706 and step S707 may be performed simultaneously.

In this embodiment of this application, the health detection device may divide to-be-transmitted data into a plurality of user health data sets based on detection time points. Detection time points of user health data in different user health data sets are within different time intervals, and the user health data in the different time intervals may be sequentially transmitted based on an importance order of the user health data in the different time intervals. The terminal device may output user health data and/or a related health indicator that are/is in a time interval and that have/has relatively high importance without waiting for a long time. In this way, the user can view the user health data and/or the related health indicator that are/is in the time interval and that have/has relatively high importance as soon as possible, thereby effectively improving user experience. For example, when the importance of the user health data in the first time interval is higher than the importance of the user health data in the second time interval, the health detection device may preferentially transmit some data in the first time interval (that is, the first transmission data in the first user health data set), and then transmit some data in the second time interval (that is, the second transmission data in the second user health data set). The terminal device may output the user health data and/or the related health indicator that are/is in the first time interval and that have/has relatively high importance without waiting for a long time, so that the user can view the user health data and/or a related health indicator that are/is in the first time interval and that have/has relatively high importance as soon as possible, thereby effectively improving user experience.

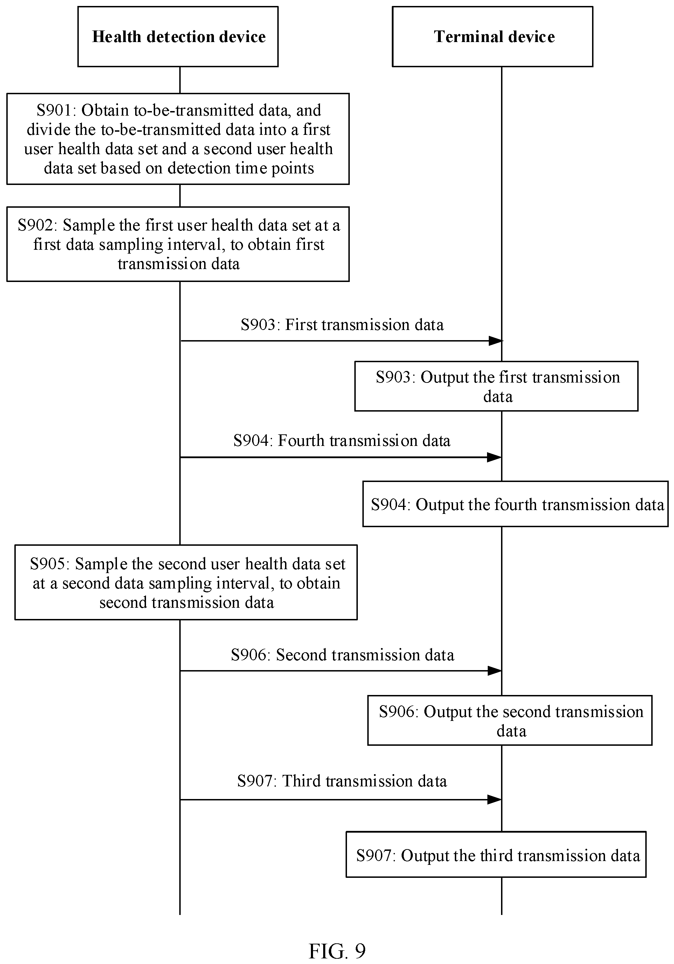

FIG. 9 is a schematic flowchart of still another data transmission method. In comparison with the data transmission method in FIG. 7, in the data transmission method in FIG. 9, when transmission of all user health data that is in a time interval and that has relatively high importance is completed, user health data that is in a time interval and that has relatively low importance may be further transmitted. As shown in FIG. 9, the method includes but is not limited to S901 to S907.

S901: A health detection device obtains to-be-transmitted data, and divides the to-be-transmitted data into a first user health data set and a second user health data set based on detection time points.

S902: The health detection device samples the first user health data set at a first data sampling interval, to obtain first transmission data, where the first data sampling interval is determined based on a data amount of the first user health data set, a data transmission rate, and expected transmission waiting duration.

S903: The health detection device transmits the first transmission data to a terminal device, and when receiving the first transmission data from the health detection device, the terminal device outputs the first transmission data.

S904: The health detection device transmits fourth transmission data to the terminal device, and when receiving the fourth transmission data from the health detection device, the terminal device outputs the fourth transmission data. The fourth transmission data is a part or all of the first user health data set except the first transmission data.

S905: The health detection device samples the second user health data set at a second data sampling interval, to obtain second transmission data, where the second data sampling interval is determined based on a data amount of the second user health data set, the data transmission rate, and the expected transmission waiting duration.

S906: The health detection device transmits the second transmission data to the terminal device, and when receiving the second transmission data from the health detection device, the terminal device outputs the second transmission data.

S907: The health detection device transmits third transmission data to the terminal device, and when receiving the third transmission data from the health detection device, the terminal device outputs the third transmission data. The third transmission data is a part or all of the second user health data set except the second transmission data.

It should be noted that for related descriptions of step S901 to step S907, refer to the related embodiment in FIG. 3 or FIG. 7. Details are not described again.

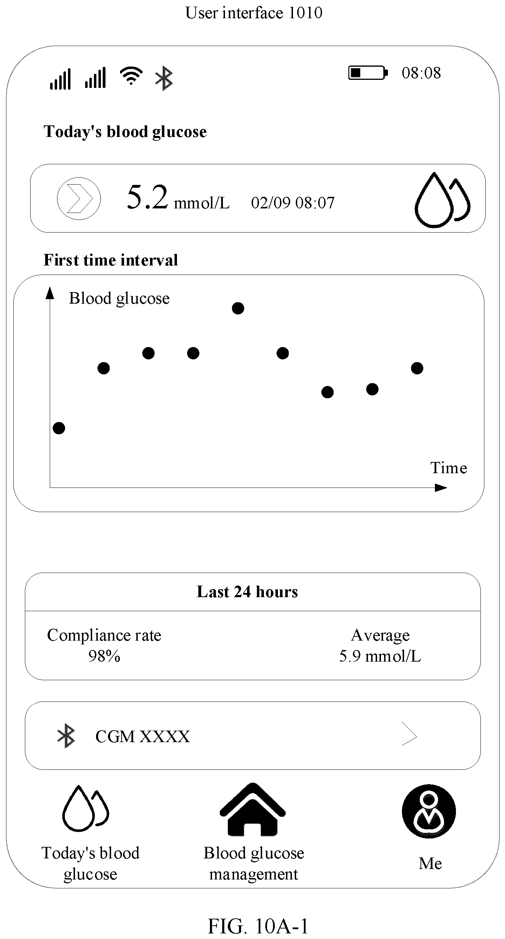

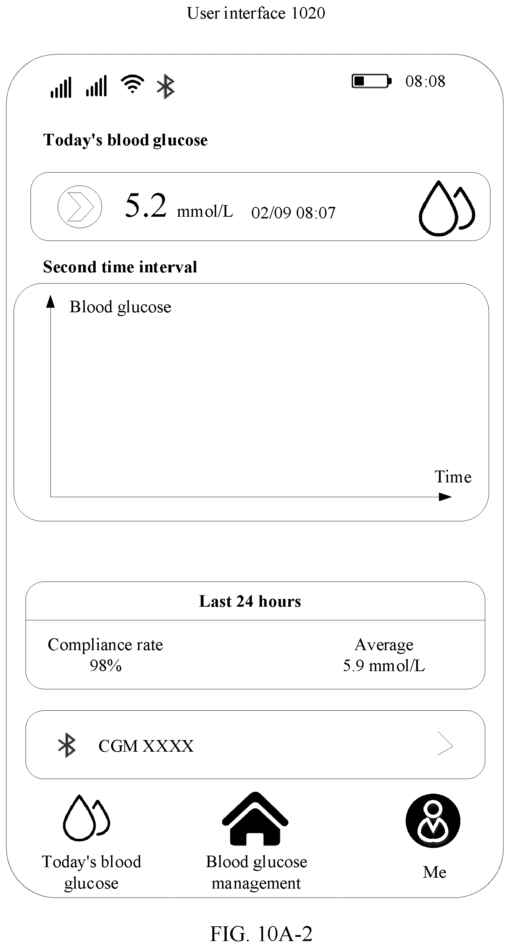

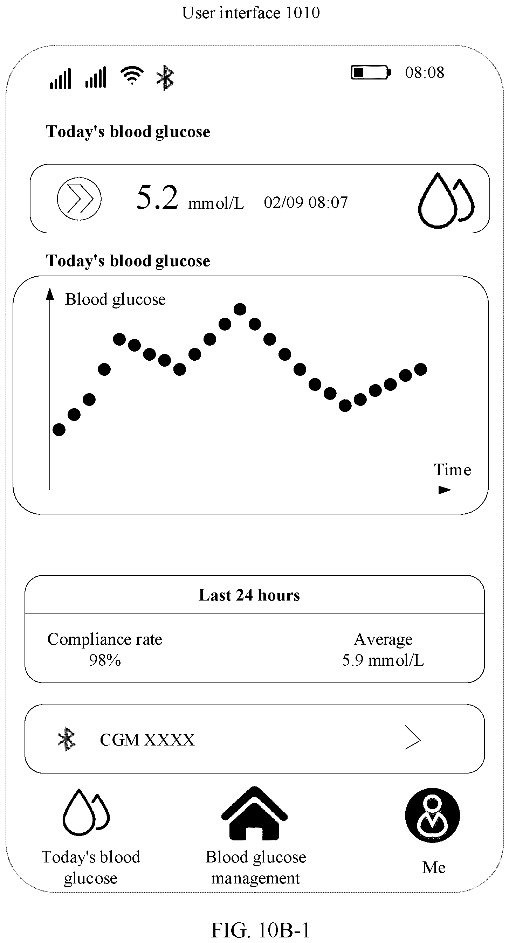

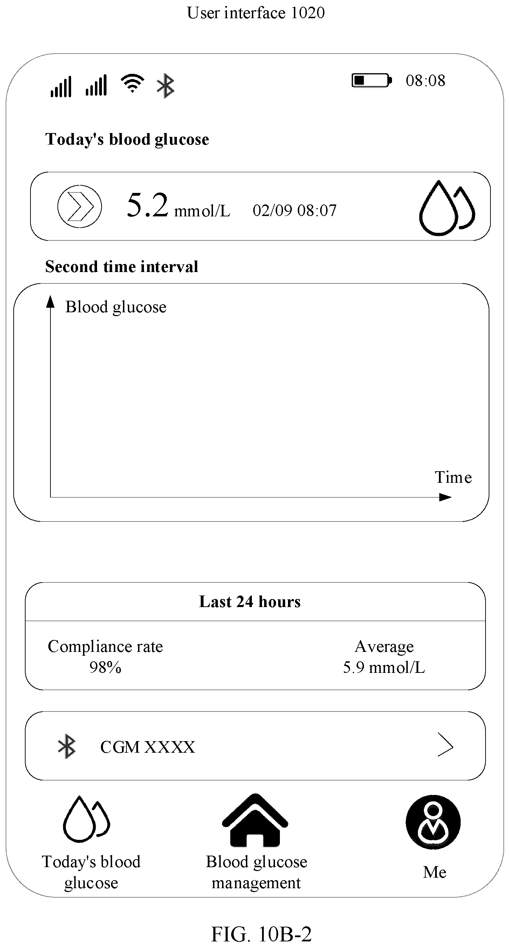

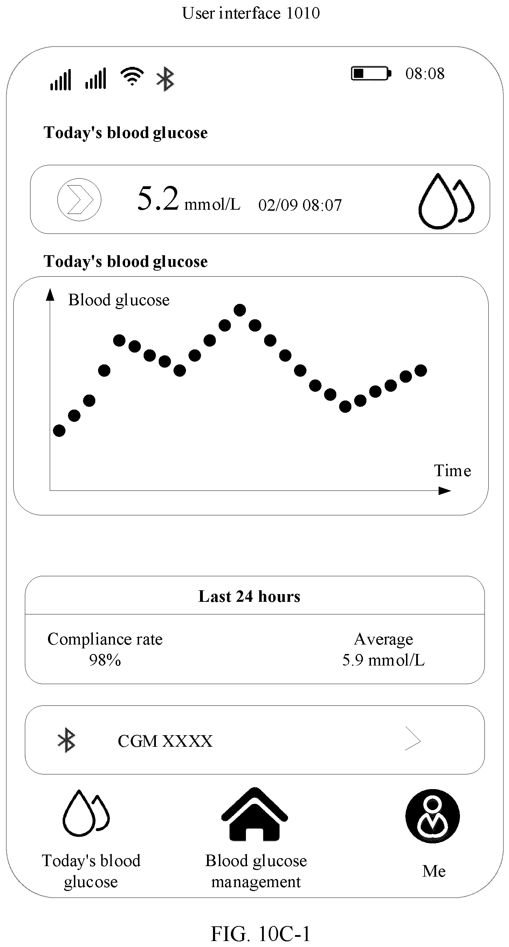

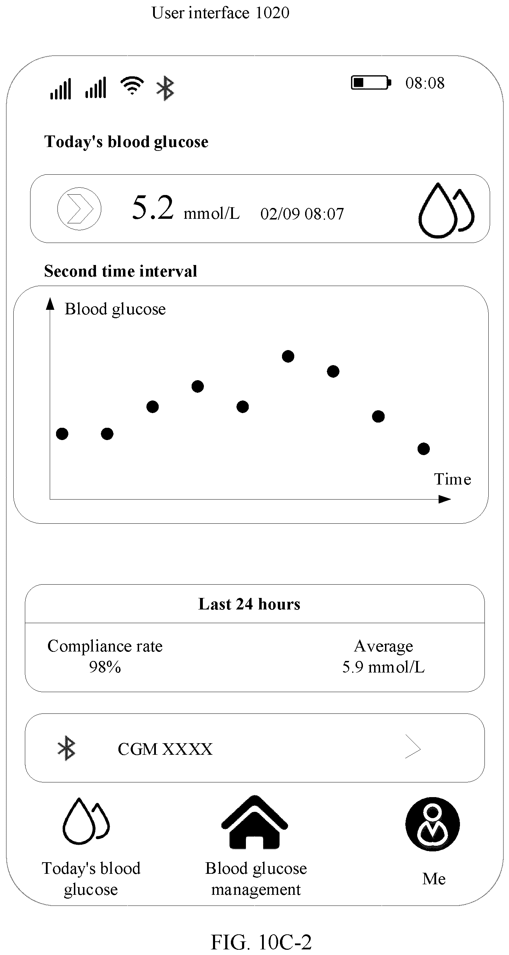

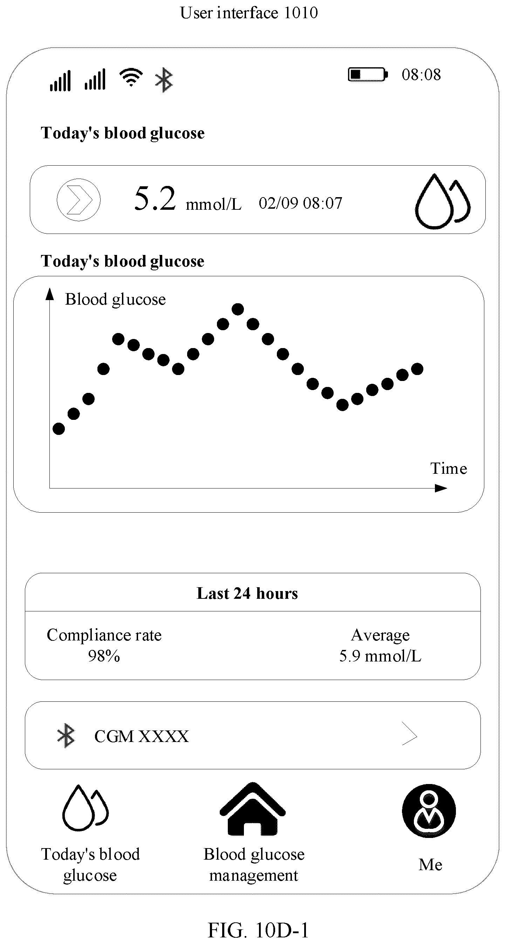

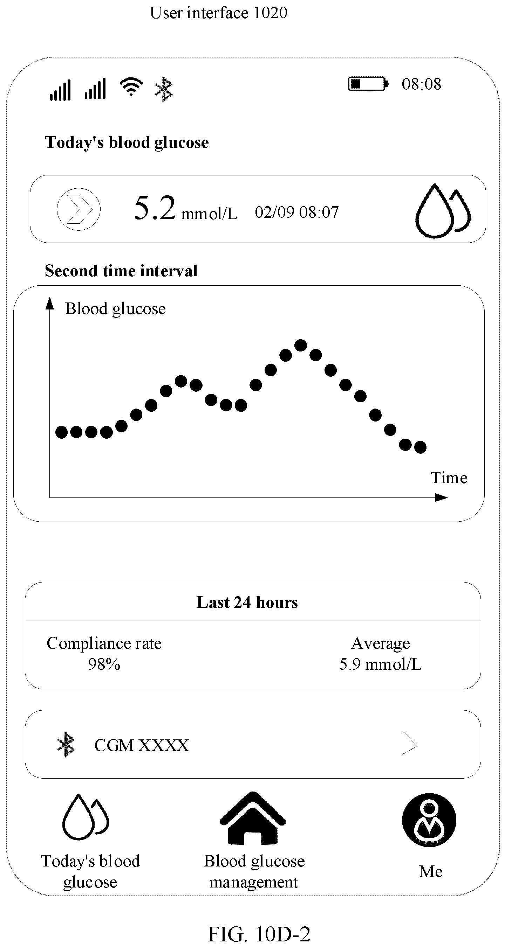

The following describes the embodiment in FIG. 9 in detail with reference to user interfaces in FIG. 10A-1 to FIG. 10D-2 by using an example in which the user health data is blood glucose data.

As shown in FIG. 10A-1 and FIG. 10A-2, when transmission of the first transmission data in the first user health data set is completed, the terminal device may sequentially output, in a user interface 1010, nine pieces of blood glucose data whose detection time points are within the first time interval (that is, preferentially transmitted blood glucose data in the first time interval). In this case, blood glucose data whose detection time point is within the second time interval has not been output in a user interface 1020.

As shown in FIG. 10B-1 and FIG. 10B-2, when transmission of all data in the first user health data set is completed, the terminal device may sequentially output, in a user interface 1010, 25 pieces of blood glucose data whose detection time points are within the first time interval (that is, all blood glucose data in the first time interval). In this case, blood glucose data whose detection time point is within the second time interval has not been output in a user interface 1020.

As shown in FIG. 10C-1 and FIG. 10C-2, when transmission of all data in the first user health data set is completed, and transmission of the second transmission data in the second user health data set is completed, the terminal device may sequentially output, in a user interface 1010, 25 pieces of blood glucose data whose detection time points are within the first time interval (that is, all blood glucose data in the first time interval). In this case, nine pieces of blood glucose data whose detection time points are within the second time interval (that is, preferentially transmitted blood glucose data in the second time interval) are output in a user interface 1020.

As shown in FIG. 10D-1 and FIG. 10D-2, when transmission of all data in the first user health data set is completed, and transmission of all data in the second user health data set is completed, the terminal device may sequentially output, in a user interface 1010, 25 pieces of blood glucose data whose detection time points are within the first time interval (that is, all blood glucose data in the first time interval). In this case, 25 pieces of blood glucose data whose detection time points are within the second time interval (that is, all blood glucose data in the second time interval) may be output in a user interface 1020.

In this embodiment of this application, the health detection device may divide to-be-transmitted data into a plurality of user health data sets based on detection time points. Detection time points of user health data in the different user health data sets are within different time intervals, and the user health data in the different time intervals may be sequentially transmitted based on an importance order of the user health data in the different time intervals. The terminal device may output all user health data and/or related health indicators that are in a time interval and that have relatively high importance without waiting for a long time. In this way, the user can view all the user health data and/or the related health indicators that are in the time interval and that have relatively high importance as soon as possible, thereby effectively improving user experience. For example, when importance of user health data in a first time interval is higher than importance of user health data in a second time interval, the health detection device may output all user health data and/or related health data in the first time interval without waiting for a long time, and then transmit all user health data and/or related health data in the second time interval. In this way, the user can view all the user health data and/or the related health data that are/is in the first time interval and that have/has relatively high importance as soon as possible, thereby effectively improving user experience.

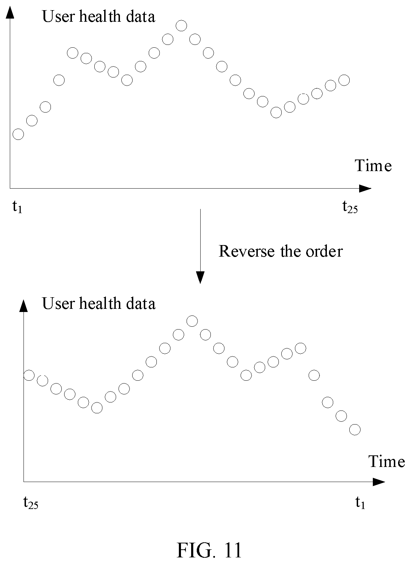

FIG. 11 is a diagram of yet another data transmission method. In comparison with the data transmission method in FIG. 3, the data transmission method in FIG. 7, and the data transmission method in FIG. 9, in the data transmission method in FIG. 11, user health data in a user health data set may alternatively be transmitted in a reversed order. As shown in FIG. 11, white circles represent user health data detected at 25 detection time points in a first user health data set, and the detection time points corresponding to the 25 pieces of user health data are respectively t1, t2, ..., t24, and t25. After a health detection device reverses an order of the user health data in the first user health data set based on a sequence of the detection time points, a reversed-order first user health data set may be obtained, the detection time points corresponding to the 25 pieces of user health data in the reversed-order first user health data set are respectively t25, t24, ..., t2, and t1. The health detection device may sequentially transmit the user health data in the reversed-order first user health data set.

Optionally, when the data is transmitted in a reversed order, as shown in FIG. 2(b), a terminal device may preferentially output blood glucose data indicated by 2022-1, then output blood glucose data indicated by 2022-2, and then output blood glucose data indicated by 2022-3.

In this embodiment of this application, the health detection device may sequentially transmit user health data in a reversed-order user health data set, and the terminal device may output user health data and/or related health data whose detection time point is close to a current moment without waiting for a long time, so that a user can view the user health data and/or the related health data whose detection time point is close to the current moment as soon as possible, thereby effectively improving user experience.

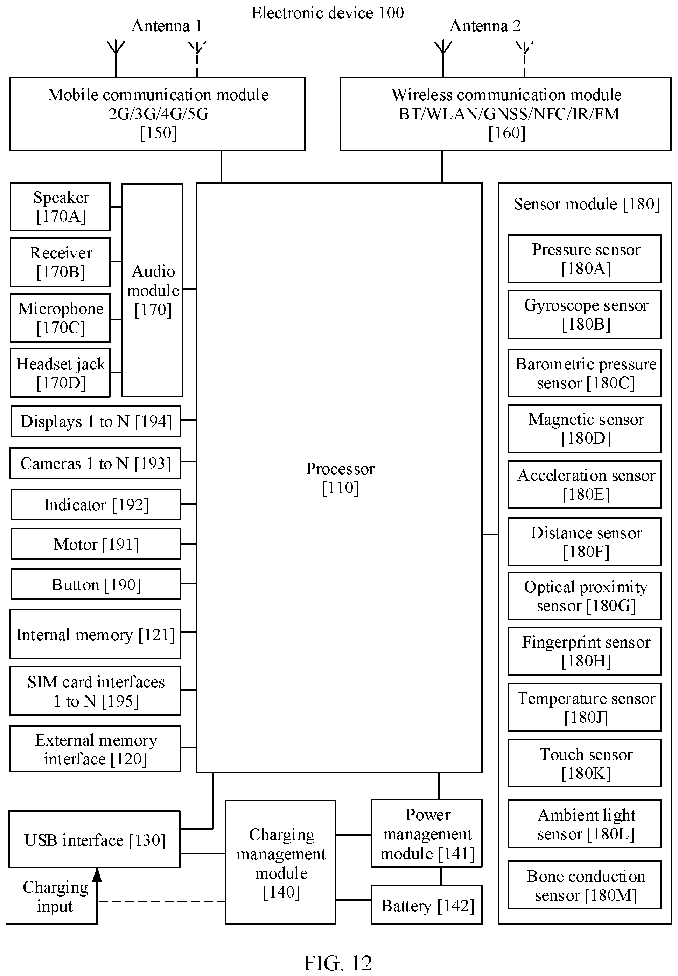

FIG. 12 is a diagram of a structure of an electronic device 100. The electronic device 100 may be the health detection device or the terminal device in the foregoing embodiment. This is not limited.

The electronic device 100 is used as an example below to describe embodiments in detail. It should be understood that the electronic device 100 shown in FIG. 12 is merely an example, and the electronic device 100 may have more or fewer components than those shown in FIG. 12, or two or more components may be combined, or there may be a different component configuration. Components shown in the figure may be implemented by hardware, software, or a combination of hardware and software that includes one or more signal processing and/or application-specific integrated circuits.

The electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identity module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in this embodiment of this application does not constitute a specific limitation on the electronic device 100. In some other embodiments of this application, the electronic device 100 may include more or fewer components than those shown in the figure, or some components may be combined, or some components may be split, or different component arrangements may be used. The components shown in the figure may be implemented by hardware, software, or a combination of software and hardware. For example, the acceleration sensor 180E and the gyroscope sensor 180B are located in smart glasses, and the camera 193, the optical proximity sensor 180G, and the display 194 are located in a smartphone.

The processor 110 may include one or more processing units. For example, the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may be a neural center and a command center of the electronic device 100. The controller may generate an operation control signal based on an instruction operation code and a time sequence signal, to complete control of instruction reading and instruction execution.

A memory may be further disposed in the processor 110, and is configured to store instructions and data. In some embodiments, the memory in the processor 110 is a cache. The memory may store instructions or data just been used or cyclically used by the processor 110. If the processor 110 needs to use the instructions or the data again, the processor 110 may directly invoke the instructions or the data from the memory. This avoids repeated access, reduces waiting time of the processor 110, thereby improving system efficiency.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/-transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The I2C interface is a two-way synchronization serial bus, and includes one serial data line (serial data line, SDA) and one serial clock line (serial clock line, SCL). In some embodiments, the processor 110 may include a plurality of groups of I2C buses. The processor 110 may be separately coupled to the touch sensor 180K, a charger, a flash, the camera 193, and the like through different I2C bus interfaces. For example, the processor 110 may be coupled to the touch sensor 180K through the I2C interface, so that the processor 110 communicates with the touch sensor 180K through the I2C bus interface, to implement a touch function of the electronic device 100.

The I2S interface may be configured to perform audio communication. In some embodiments, the processor 110 may include a plurality of groups of I2S buses. The processor 110 may be coupled to the audio module 170 through the I2S bus, to implement communication between the processor 110 and the audio module 170. In some embodiments, the audio module 170 may transmit an audio signal to the wireless communication module 160 through the I2S interface, to implement a function of answering a call through a Bluetooth headset.

The PCM interface may also be used to perform audio communication, and sample, quantize, and code an analog signal. In some embodiments, the audio module 170 may be coupled to the wireless communication module 160 through a PCM bus interface. In some embodiments, the audio module 170 may alternatively transmit an audio signal to the wireless communication module 160 through the PCM interface, to implement a function of answering a call through a Bluetooth headset. Both the I2S interface and the PCM interface may be used for audio communication.

The UART interface is a universal serial data bus, and is configured to perform asynchronous communication. The bus may be a two-way communication bus. The bus converts to-be-transmitted data between serial communication and parallel communication. In some embodiments, the UART interface is usually configured to connect the processor 110 to the wireless communication module 160. For example, the processor 110 communicates with a Bluetooth module in the wireless communication module 160 through the UART interface, to implement a Bluetooth function. In some embodiments, the audio module 170 may transmit an audio signal to the wireless communication module 160 through the UART interface, to implement a function of playing music through a Bluetooth headset.

The MIPI interface may be configured to connect the processor 110 to a peripheral component like the display 194 or the camera 193. The MIPI interface includes a camera serial interface (camera serial interface, CSI), a display serial interface (display serial interface, DSI), and the like. In some embodiments, the processor 110 communicates with the camera 193 through the CSI interface, to implement a photographing function of the electronic device 100. The processor 110 communicates with the display 194 through the DSI interface, to implement a display function of the electronic device 100.

The GPIO interface may be configured by software. The GPIO interface may be configured as a control signal or a data signal. In some embodiments, the GPIO interface may be configured to connect the processor 110 to the camera 193, the display 194, the wireless communication module 160, the audio module 170, the sensor module 180, or the like. The GPIO interface may alternatively be configured as an I2C interface, an I2S interface, a UART interface, an MIPI interface, or the like.

The USB interface 130 is an interface that conforms to a USB standard specification, and may be specifically a mini USB interface, a micro USB interface, a USB type-C interface, or the like. The USB interface 130 may be configured to connect to a charger to charge the electronic device 100, or may be configured to transmit data between the electronic device 100 and a peripheral device, or may be configured to connect to a headset for playing audio through the headset. The interface may be further configured to connect to another electronic device like an AR device.

It may be understood that an interface connection relationship between the modules shown in this embodiment of this application is merely an example for description, and does not constitute a limitation on the structure of the electronic device 100. In some other embodiments of this application, the electronic device 100 may alternatively use an interface connection manner different from that in the foregoing embodiment, or use a combination of a plurality of interface connection manners.

The charging management module 140 is configured to receive charging input from a charger. The charger may be a wireless charger or a wired charger. In some embodiments of wired charging, the charging management module 140 may receive a charging input of a wired charger through the USB interface 130. In some embodiments of wireless charging, the charging management module 140 may receive a wireless charging input through a wireless charging coil of the electronic device 100. The charging management module 140 may further supply power to the electronic device by using the power management module 141 when charging the battery 142.

The power management module 141 is configured to connect to the battery 142, the charging management module 140, and the processor 110. The power management module 141 receives an input from the battery 142 and/or the charging management module 140, and supplies power to the processor 110, the internal memory 121, an external memory, the display 194, the camera 193, the wireless communication module 160, and the like. The power management module 141 may be further configured to monitor parameters such as a battery capacity, a battery cycle count, or a battery health status (electric leakage or impedance). In some other embodiments, the power management module 141 may alternatively be disposed in the processor 110. In some other embodiments, the power management module 141 and the charging management module 140 may alternatively be disposed in a same component.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 100 may be configured to cover one or more communication frequency bands. Different antennas may be further multiplexed, to improve antenna utilization. For example, the antenna 1 may be multiplexed as a diversity antenna of a wireless local area network. In some other embodiments, the antenna may be used in combination with a tuning switch.

The mobile communication module 150 may provide a wireless communication solution that is applied to the electronic device 100 and that includes a 2G/3G/4G/5G or the like. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit a processed electromagnetic wave to the modem processor for demodulation. The mobile communication module 150 may further amplify a signal modulated by the modem processor, and convert, through the antenna 1, the signal into an electromagnetic wave for radiation. In some embodiments, at least some functional modules in the mobile communication module 150 may be disposed in the processor 110. In some embodiments, at least some functional modules of the mobile communication module 150 may be disposed in a same device as at least some modules of the processor 110.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal through an audio device (which is not limited to the speaker 170A, the receiver 170B, and the like), or displays an image or a video through the display 194. In some embodiments, the modem processor may be an independent component. In some other embodiments, the modem processor may be independent of the processor 110, and is disposed in a same component as the mobile communication module 150 or another functional module.

The wireless communication module 160 may provide a wireless communication solution that is applied to the electronic device 100 and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more components integrating at least one communication processing module. The wireless communication module 160 receives an electromagnetic wave through the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert, through the antenna 2, the signal into an electromagnetic wave for radiation.

In some embodiments, the antenna 1 and the mobile communication module 150 of the electronic device 100 are coupled, and the antenna 2 and the wireless communication module 160 of the electronic device 100 are coupled, so that the electronic device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation system, SBAS).

The electronic device 100 implements a display function by using the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs, and the one or more GPUs execute program instructions to generate or change display information.

The display 194 is configured to display an image, a video, and the like. The display 194 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini-LED, a micro-LED, a micro-OLED, a quantum dot light-emitting diode (quantum dot light-emitting diode, QLED), or the like. In some embodiments, the electronic device 100 may include one or N displays 194, where N is a positive integer greater than 1.

The electronic device 100 can implement a photographing function by using the ISP, the camera 193, the video codec, the GPU, the display 194, the application processor, and the like.

The ISP is configured to process data fed back by the camera 193. For example, during photographing, a shutter is pressed, light is transmitted to a photosensitive element of the camera through a lens, an optical signal is converted into an electrical signal, and the photosensitive element of the camera transmits the electrical signal to the ISP for processing, to convert the electrical signal into a visible image. The ISP may further perform algorithm optimization on noise, and brightness of the image. The ISP may further optimize parameters such as exposure and a color temperature of a photographing scenario. In some embodiments, the ISP may be disposed in the camera 193.

The camera 193 is configured to capture a static image or a video. An optical image of an object is generated through the lens, and is projected onto the photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP to convert the electrical signal into a digital image signal. The ISP outputs the digital image signal to the DSP for processing. The DSP converts the digital image signal into an image signal in a standard format like RGB or YUV. In some embodiments, the electronic device 100 may include one or N cameras 193, where N is a positive integer greater than 1.

The digital signal processor is configured to process a digital signal, and may process another digital signal in addition to the digital image signal. For example, when the electronic device 100 selects a frequency, the digital signal processor is configured to perform Fourier transform on frequency energy.

The video codec is configured to compress or decompress a digital video. The electronic device 100 may support one or more video codecs. Therefore, the electronic device 100 may play or record videos in a plurality of coding formats, for example, moving picture experts group (moving picture experts group, MPEG)-1, MPEG-2, MPEG-3, and MPEG-4.

The NPU is a neural-network (neural-network, NN) computing processor, and simulates a biological neural network structure such as a transmission mode between neurons in a human brain to perform rapid processing on input information, and can perform continuous self-learning. Applications such as intelligent cognition of the electronic device 100 may be implemented through the NPU, for example, image recognition, facial recognition, speech recognition, and text understanding.

The external memory interface 120 may be used to connect to an external storage card, for example, a micro SD card, to extend a storage capability of the electronic device 100. The external storage card communicates with the processor 110 through the external memory interface 120, to implement a data storage function. For example, files such as music and videos are stored in the external storage card.

The internal memory 121 may be configured to store computer-executable program code. The executable program code includes instructions. The processor 110 runs the instructions stored in the internal memory 121, to perform various functional applications of the electronic device 100 and data processing. The internal memory 121 may include a program storage area and a data storage area. The program storage area may store an operating system, an application required by at least one function (for example, a sound playing function or an image playing function), and the like. The data storage area may store data (for example, audio data and a phone book) created in a process of using the electronic device 100, and the like. In addition, the internal memory 121 may include a high-speed random access memory, or may further include a nonvolatile memory, for example, at least one magnetic disk storage device, a flash memory, or a universal flash storage (universal flash storage, UFS).

The electronic device 100 may implement an audio function, for example, music playing and recording, by using the audio module 170, the speaker 170A, the receiver 170B, the microphone 170C, the headset jack 170D, the application processor, and the like.

The audio module 170 is configured to convert digital audio information into an analog audio signal for output, and is further configured to convert an analog audio input into a digital audio signal. The audio module 170 may be further configured to encode and decode an audio signal. In some embodiments, the audio module 170 may be disposed in the processor 110, or some functional modules in the audio module 170 are disposed in the processor 110.

The speaker 170A, also referred to as a "horn", is configured to convert an electrical audio signal into a sound signal. The electronic device 100 may be used to listen to music or answer a hands-free call through the speaker 170A. Optionally, the speaker 170A may be configured to output interaction guide information (for example, first interaction guide information, second interaction guide information, third interaction guide information, fourth interaction guide information, and fifth interaction guide information).

The receiver 170B, also referred to as an "earpiece", is configured to convert an electrical audio signal into a sound signal. When a call is answered or speech information is received through the electronic device 100, the receiver 170B may be put close to a human ear to listen to a voice.

The microphone 170C, also referred to as a "mike" or a "mic", is configured to convert a sound signal into an electrical signal. When making a call or sending voice information, a user may make a sound near the microphone 170C through the mouth of the user, to input a sound signal to the microphone 170C. At least one microphone 170C may be disposed in the electronic device 100. In some other embodiments, two microphones 170C may be disposed in the electronic device 100, to collect a sound signal and further implement a noise reduction function. In some other embodiments, three, four, or more microphones 170C may alternatively be disposed in the electronic device 100, to collect a sound signal, implement noise reduction, identify a sound source, implement a directional recording function, and the like.

The headset jack 170D is configured to connect to a wired headset. The headset jack 170D may be the USB interface 130, or may be a 3.5 mm open mobile terminal platform (open mobile terminal platform, OMTP) standard interface, or the like.

The pressure sensor 180A is configured to sense a pressure signal, and can convert the pressure signal into an electrical signal. In some embodiments, the pressure sensor 180A may be disposed on the display 194. There are a plurality of types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 100 determines pressure intensity based on the change in the capacitance. When a touch operation is performed on the display 194, the electronic device 100 detects intensity of the touch operation through the pressure sensor 180A. The electronic device 100 may further calculate a touch position based on a detection signal of the pressure sensor 180A. In some embodiments, touch operations that are performed in a same touch position but have different touch operation intensity may correspond to different operation instructions. For example, when a touch operation whose touch operation intensity is less than a first pressure threshold is performed on an SMS message application icon, an instruction for viewing an SMS message is performed. When a touch operation whose touch operation intensity is greater than or equal to the first pressure threshold is performed on the SMS message application icon, an instruction for creating a new SMS message is executed.

The gyroscope sensor 180B may be configured to determine a motion pose of the electronic device 100. In some embodiments, an angular velocity of the electronic device 100 around three axes (that is, axes x, y, and z) may be determined by using the gyroscope sensor 180B. The gyroscope sensor 180B may be configured to implement image stabilization during photographing. For example, when the shutter is pressed, the gyroscope sensor 180B detects an angle at which the electronic device 100 jitters, obtains, through calculation based on the angle, a distance for which a lens module needs to compensate, and allows the lens to cancel the jitter of the electronic device 100 through reverse motion, to implement image stabilization. The gyroscope sensor 180B may be further used in a navigation scenario and a motion-sensing game scenario.

Optionally, the gyroscope sensor 180B may be configured to collect angular velocity data when a to-be-measured person performs a fourth action.

The barometric pressure sensor 180C is configured to measure barometric pressure. In some embodiments, the electronic device 100 calculates an altitude through the barometric pressure measured by the barometric pressure sensor 180C, to assist in positioning and navigation.

The magnetic sensor 180D includes a Hall effect sensor. The electronic device 100 may detect opening and closing of a flip cover by using the magnetic sensor 180D. In some embodiments, when the electronic device 100 is a flip device, the electronic device 100 may detect opening and closing of a flip cover by using the magnetic sensor 180D. Further, a feature such as automatic unlocking upon opening of the flip cover is set based on a detected opening or closing state of the flip cover.

The acceleration sensor 180E may detect magnitudes of accelerations of the electronic device 100 in various directions (usually on three axes). When the electronic device 100 is still, a magnitude and a direction of gravity may be detected. The acceleration sensor 180E may be further configured to identify a pose of the electronic device, and is used in an application such as a pedometer or switching between a landscape mode and a portrait mode.

Optionally, the acceleration sensor 180E may be configured to collect acceleration data when the to-be-measured person keeps a first action.

The distance sensor 180F is configured to measure a distance. The electronic device 100 may measure the distance in an infrared manner or a laser manner. In some embodiments, in a photographing scenario, the electronic device 100 may measure a distance by using the distance sensor 180F, to implement quick focusing.

The optical proximity sensor 180G may include, for example, a light-emitting diode (LED) and an optical detector, for example, a photodiode. The light-emitting diode may be an infrared light-emitting diode. The electronic device 100 emits infrared light through the light-emitting diode. The electronic device 100 detects infrared reflected light from a nearby object through the photodiode. When sufficient reflected light is detected, it may be determined that there is an object near the electronic device 100. When insufficient reflected light is detected, the electronic device 100 may determine that there is no object near the electronic device 100. The electronic device 100 may detect, by using the optical proximity sensor 180G, that a user holds the electronic device 100 close to an ear for a call, to automatically perform screen-off for power saving. The optical proximity sensor 180G may further be used for automatically unlocking or locking a screen in leather case mode or pocket mode.

The ambient light sensor 180L is configured to sense ambient light brightness. The electronic device 100 may adaptively adjust brightness of the display 194 based on the sensed ambient light brightness. The ambient light sensor 180L may further be configured to automatically adjust white balance during photographing. The ambient light sensor 180L may further cooperate with the optical proximity sensor 180G to detect whether the electronic device 100 is in a pocket, to avoid an accidental touch.

The fingerprint sensor 180H is configured to collect a fingerprint. The electronic device 100 may use a feature of the collected fingerprint to implement fingerprint-based unlocking, application lock access, fingerprint-based photographing, fingerprint-based call answering, and the like.

The temperature sensor 180J is configured to detect a temperature. In some embodiments, the electronic device 100 executes a temperature processing policy based on the temperature detected by the temperature sensor 180J. For example, when the temperature reported by the temperature sensor 180J exceeds a threshold, the electronic device 100 lowers performance of a processor nearby the temperature sensor 180J, to reduce power consumption for thermal protection. In some other embodiments, when the temperature is less than another threshold, the electronic device 100 heats the battery 142, to avoid an abnormal shutdown of the electronic device 100 caused by a low temperature. In some other embodiments, when the temperature is less than still another threshold, the electronic device 100 boosts an output voltage of the battery 142, to avoid an abnormal shutdown caused by a low temperature.

The touch sensor 180K is also referred to as a "touch panel". The touch sensor 180K may be disposed on the display 194, and the touch sensor 180K and the display 194 constitute a touchscreen, which is also referred to as a "touch screen". The touch sensor 180K is configured to detect a touch operation performed on or near the touch sensor. The touch sensor may transfer the detected touch operation to the application processor, to determine a type of a touch event. A visual output related to the touch operation may be provided through the display 194. In some other embodiments, the touch sensor 180K may alternatively be disposed on a surface of the electronic device 100 at a position different from that of the display 194.

The bone conduction sensor 180M may obtain a vibration signal. In some embodiments, the bone conduction sensor 180M may obtain a vibration signal of a vibration bone of a human vocal-cord part. The bone conduction sensor 180M may further be in contact with a human pulse, to receive a blood pressure beating signal. In some embodiments, the bone conduction sensor 180M may alternatively be disposed in the headset, to obtain a bone conduction headset. The audio module 170 may obtain a voice signal through parsing based on the vibration signal that is of the vibration bone of the vocal-cord part and that is obtained by the bone conduction sensor 180M, to implement a voice function. The application processor may parse heart rate information based on the blood pressure beating signal obtained by the bone conduction sensor 180M, to implement a heart rate detection function.

The button 190 includes a power button, a volume button, and the like. The button 190 may be a mechanical button, or may be a touch button. The electronic device 100 may receive a button input, and generate a button signal input related to a user setting and function control of the electronic device 100.

The motor 191 may generate a vibration prompt. The motor 191 may be configured to provide an incoming call vibration prompt or a touch vibration feedback. For example, touch operations performed on different applications (for example, photographing and audio playing) may correspond to different vibration feedback effect. The motor 191 may further correspond to different vibration feedback effect for touch operations performed on different areas of the display 194. Different application scenarios (for example, a time reminder, information receiving, an alarm clock, and a game) may also correspond to different vibration feedback effect. Touch vibration feedback effect may alternatively be customized.

The indicator 192 may be an indicator light, and may be configured to indicate a charging status and a power change, or may be configured to indicate a message, a missed call, a notification, and the like.

The SIM card interface 195 is configured to connect to a SIM card. The SIM card may be inserted into the SIM card interface 195 or removed from the SIM card interface 195, to implement contact with or separation from the electronic device 100. The electronic device 100 may support one or N SIM card interfaces, where N is a positive integer greater than 1. The SIM card interface 195 may support a nano-SIM card, a micro-SIM card, a SIM card, and the like. A plurality of cards may be inserted into a same SIM card interface 195 at the same time. The plurality of cards may be of a same type or different types. The SIM card interface 195 may be compatible with different types of SIM cards. The SIM card interface 195 is also compatible with an external storage card. The electronic device 100 interacts with a network through the SIM card, to implement functions such as conversation and data communication. In some embodiments, the electronic device 100 uses an eSIM, that is, an embedded SIM card. The eSIM card may be embedded into the electronic device 100, and cannot be separated from the electronic device 100.

A software system of the electronic device 100 may use a layered architecture, an event-driven architecture, a microkernel architecture, a micro service architecture, or a cloud architecture. In an embodiment of this application, an Android system with a layered architecture is used as an example to describe a software structure of the electronic device 100.

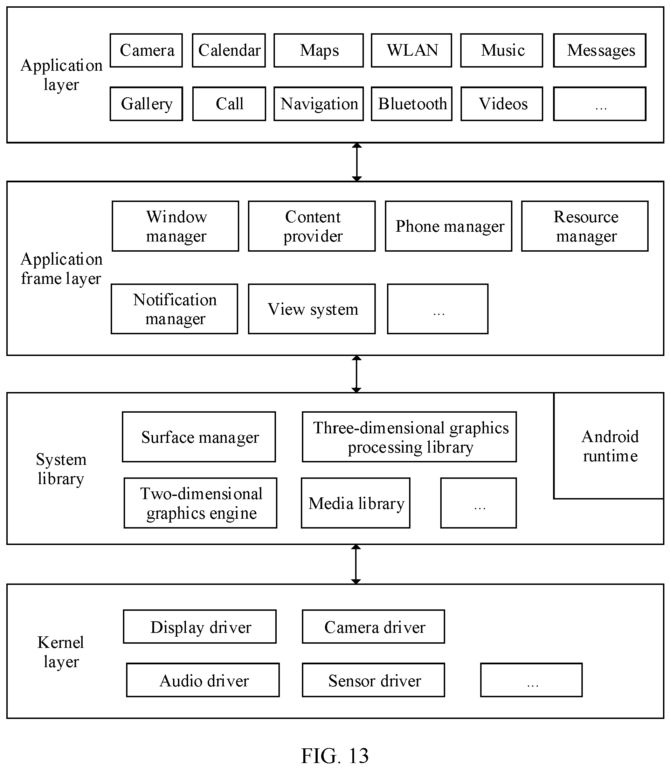

FIG. 13 is a block diagram of the software structure of the electronic device 100 according to an embodiment of this application.

In a layered architecture, software is divided into several layers, and each layer has a clear role and task. The layers communicate with each other through a software interface. In some embodiments, the Android system is divided into four layers: an application layer, an application framework layer, an Android runtime (Android runtime) and a system library, and a kernel layer.

The application layer may include a series of application packages.

As shown in FIG. 13, the application packages may include applications such as Camera, Gallery, Calendar, Call, Maps, Navigation, WLAN, Bluetooth, Music, Videos, and Messages.

The application framework layer provides an application programming interface (application programming interface, API) and a programming framework for an application at the application layer. The application framework layer includes some predefined functions.

As shown in FIG. 13, the application framework layer may include a window manager, a content provider, a view system, a phone manager, a resource manager, a notification manager, and the like.

The window manager is configured to manage a window program. The window manager may obtain a size of a display, determine whether there is a status bar, perform screen locking, take a screenshot, and the like.

The content provider is configured to: store and obtain data, and enable the data to be accessed by an application. The data may include videos, images, audio, calls that are made and answered, browsing histories and bookmarks, phone books, and the like.

The view system includes visual controls such as a control for displaying a text and a control for displaying an image. The view system may be configured to construct an application. A display interface may include one or more views. For example, a display interface including an SMS message notification icon may include a text display view and an image display view.

The phone manager is configured to provide a communication function of the electronic device 100, for example, management of a call status (including answering, declining, or the like).

The resource manager provides various resources for an application, such as a localized character string, an icon, a picture, a layout file, and a video file.

The notification manager enables an application to display notification information in a status bar, and may be configured to convey a notification type message. The message may automatically disappear after a short stay without a user interaction. For example, the notification manager is configured to notify download completion, give a message notification, and the like. The notification manager may alternatively be a notification that appears in a top status bar of the system in a form of a graph or a scroll bar text, for example, a notification of an application running in the background, or may be a notification that appears on the screen in a form of a dialog window. For example, text information is prompted in the status bar, an alert tone is made, the electronic device vibrates, or an indicator light blinks.

The Android runtime includes a kernel library and a virtual machine. The Android runtime is responsible for scheduling and management of the Android system.

The core library includes two parts: One part is a function that needs to be invoked by Java language, and the other part is a core library of Android.

The application layer and the application framework layer run in the virtual machine. The virtual machine executes Java files at the application layer and the application framework layer as binary files. The virtual machine is configured to perform functions such as object lifecycle management, stack management, thread management, security and exception management, and garbage collection.

The system library may include a plurality of functional modules, for example, a surface manager (surface manager), a media library (Media Library), a three-dimensional graphics processing library (for example, OpenGL ES), and a two-dimensional graphics engine (for example, SGL).

The surface manager is configured to manage a display subsystem and provide fusion of 2D and 3D layers for a plurality of applications.

The media library supports playback and recording in a plurality of commonly used audio and video formats, static image files, and the like. The media library may support a plurality of audio and video coding formats such as MPEG-4, H.264, MP3, AAC, AMR, JPG, and PNG.

The three-dimensional graphics processing library is configured to implement three-dimensional graphics drawing, image rendering, composition, layer processing, and the like.

The two-dimensional graphics engine is a drawing engine for two-dimensional drawing.

The kernel layer is a layer between hardware and software. The kernel layer includes at least a display driver, a camera driver, an audio driver, and a sensor driver.

In conclusion, the foregoing embodiments are merely intended for describing the technical solutions of this application, but not for limiting this application. Although this application is described in detail with reference to the foregoing embodiments, persons of ordinary skill in the art should understand that they may still make modifications to the technical solutions described in the foregoing embodiments or make equivalent replacements to some technical features thereof, without departing from the scope of the technical solutions of embodiments of this application.

**Claims**

1. A data transmission method, wherein the method comprises:

    obtaining a first user health data set, wherein the first user health data set comprises user health data detected at a plurality of detection time points;
    sampling the to-be-transmitted first user health data set at a first data sampling interval, to obtain first transmission data, wherein the first data sampling interval is determined based on a data amount of the first user health data set, a data transmission rate, and expected transmission waiting duration; and
    transmitting the first transmission data to a terminal device.

2. The method according to claim 1, wherein
   detection time points of all pieces of user health data in the first user health data set are within a first time interval.

3. The method according to claim 2, wherein the first time interval is a time interval of last 24 hours.

4. The method according to claim 2 or 3, wherein the method further comprises:

    sampling a second user health data set at a second data sampling interval, to obtain second transmission data, wherein the second user health data set comprises a plurality of pieces of user health data whose detection time points are outside the first time interval, and the second data sampling interval is determined based on a data amount of the second user health data set, the data transmission rate, and the expected transmission waiting duration; and
    transmitting the second transmission data to the terminal device.

5. The method according to claim 4, wherein
   detection time points of all pieces of user health data in the second user health data set are within a second time interval.

6. The method according to claim 5, wherein the second time interval is a time interval between last 48 hours and last 24 hours.

7. The method according to any one of claims 4 to 6, wherein after transmitting the second transmission data to the terminal device, the method further comprises:
   transmitting third transmission data to the terminal device, wherein the third transmission data is a part or all of the second user health data set except the second transmission data.

8. The method according to any one of claims 1 to 7, wherein after transmitting the first transmission data to the terminal device, the method further comprises:
   transmitting fourth transmission data to the terminal device, wherein the fourth transmission data is a part or all of the first user health data set except the first transmission data.

9. The method according to any one of claims 1 to 8, wherein the first transmission data comprises user health data detected last time.

10. The method according to any one of claims 1 to 9, wherein the method further comprises:
    receiving the expected transmission waiting duration from the terminal device.

11. A data transmission method, wherein the method comprises:

    receiving first transmission data from a health detection device, wherein the first transmission data is obtained by the health detection device by sampling a first user health data set at a first data sampling interval, the first user health data set comprises user health data detected at a plurality of detection time points, and the first data sampling interval is determined based on a data amount of the first user health data set, a data transmission rate, and expected transmission waiting duration; and

outputting the first transmission data.

12. The method according to claim 11, wherein
detection time points of all pieces of user health data in the first transmission data are within a first time interval.

13. The method according to claim 12, wherein the first time interval is a time interval of last 24 hours.

14. The method according to claim 12 or 13, wherein the method further comprises:

receiving second transmission data from the health detection device, wherein the second transmission data is obtained by the health detection device by sampling a second user health data set at a second data sampling interval, the second user health data set comprises a plurality of pieces of user health data whose detection time points are outside the first time interval, and the second data sampling interval is determined based on a data amount of the second user health data set, the data transmission rate, and the expected transmission waiting duration; and

outputting the second transmission data.

15. The method according to claim 14, wherein
detection time points of all pieces of user health data in the second transmission data are within a second time interval.

16. The method according to claim 15, wherein the second time interval is a time interval between last 48 hours and last 24 hours.

17. The method according to any one of claims 14 to 16, wherein after receiving the second transmission data from the health detection device, the method further comprises:

receiving third transmission data from the health detection device, wherein the third transmission data is a part or all of the second user health data set except the second transmission data; and
outputting the third transmission data.

18. The method according to any one of claims 11 to 17, wherein after receiving the first transmission data from the health detection device, the method further comprises:

receiving fourth transmission data from the health detection device, wherein the fourth transmission data is a part or all of the first user health data set except the first transmission data; and
outputting the fourth transmission data.

19. The method according to any one of claims 11 to 18, wherein the first transmission data comprises user health data detected last time.

20. The method according to any one of claims 11 to 19, wherein the method further comprises:
sending the expected transmission waiting duration to the health detection device.

21. A health detection device, comprising a memory, one or more processors, a plurality of applications, and one or more programs, wherein the one or more programs are stored in the memory; and when the one or more processors execute the one or more programs, the health detection device is enabled to implement the method according to any one of claims 1 to 10.

22. A terminal device, comprising a touchscreen, a memory, one or more processors, a plurality of applications, and one or more programs, wherein the one or more programs are stored in the memory; and when the one or more processors execute the one or more programs, the terminal device is enabled to implement the method according to any one of claims 11 to 20.

23. A computer storage medium, comprising computer instructions, wherein when the computer instructions are run on a health detection device, the health detection device is enabled to perform the method according to any one of claims 1 to 10, or when the computer instructions are run on a terminal device, the terminal device is enabled to perform the method according to any one of claims 11 to 20.

24. A computer program product, wherein when the computer program product runs on a computer, the computer is enabled to perform the method according to any one of claims 1 to 10 or the method according to any one of claims 11 to 20.

FIG. 1

FIG. 2(a)

FIG. 2(b)

| Health detection device | Terminal device |

S301: Obtain a first user health data set

S302: Sample the to-be-transmitted first user health data set at a first data sampling interval, to obtain first transmission data

S303: First transmission data

S304: Output the first transmission data

FIG. 3

FIG. 4A

User health data

Time

Sample at an interval of $S_1=3$

User health data

Time

FIG. 4B

User health data

Time

Sample at an interval of $S_1=3$

User health data

Time

FIG. 4C

**Today's blood glucose**

5.2 mmol/L   02/09 08:07

**Today's blood glucose**

Blood glucose

Time

| Last 24 hours | |
| --- | --- |
| Compliance rate 98% | Average 5.9 mmol/L |

CGM XXXX   Transmission task in the queue...   — 501

Today's blood glucose

Blood glucose management

Me

FIG. 5A

FIG. 5B

**Today's blood glucose**

5.2 mmol/L    02/09 08:07

**Today's blood glucose**

Blood glucose

Time

**Last 24 hours**

Compliance rate          Average
98%                5.9 mmol/L

501

CGM XXXX          Transmission is completed

Today's blood
glucose

Blood glucose
management

Me

FIG. 5C

Today's blood glucose

5.2 mmol/L  02/09 08:07

Today's blood glucose

Blood glucose

Time

**Last 24 hours**

Compliance rate
98%

Average
5.9 mmol/L

CGM XXXX          Transmission fails          501

Today's blood
glucose

Blood glucose
management

Me

FIG. 5D

Today's blood glucose

5.2 mmol/L   02/09 08:07

Today's blood glucose

Blood glucose

Time

| Last 24 hours | |
|---|---|
| Compliance rate 98% | Average 5.9 mmol/L |

501

| CGM XXXX | Transmitting: 20% |
|---|---|

Today's blood glucose

Blood glucose management

Me

FIG. 5E

Today's blood glucose

5.2 mmol/L 02/09 08:07

Today's blood glucose — 601

Blood glucose

Time

Last 24 hours

Compliance rate
98%

Average
5.9 mmol/L

CGM XXXX

Today's blood
glucose

Blood glucose
management

Me

FIG. 6(a)

FIG. 6(b)

FIG. 6(c)

| Health detection device | Terminal device |
|---|---|

S701: Obtain to-be-transmitted data, and divide the to-be-transmitted data into a plurality of user health data sets based on detection time points, where detection time points of user health data in different user health data sets are within different time intervals

S702: Sample a first user health data set at a first data sampling interval, to obtain first transmission data

S703: First transmission data →

S703: Output the first transmission data

S704: Sample a second user health data set at a second data sampling interval, to obtain second transmission data

S705: Second transmission data →

S705: Output the second transmission data

S706: Fourth transmission data →

S706: Output the fourth transmission data

S707: Third transmission data →

S707: Output the third transmission data

FIG. 7

User interface 810

Today's blood glucose

5.2 mmol/L    02/09 08:07

First time interval

Blood glucose

Time

Last 24 hours

| Compliance rate | Average |
|---|---|
| 98% | 5.9 mmol/L |

*∦* CGM XXXX                    >

Today's blood        Blood glucose        Me
glucose              management

FIG. 8A-1

User interface 820

FIG. 8A-2

User interface 810

Today's blood glucose

5.2 mmol/L  02/09 08:07

First time interval

Blood glucose

Time

**Last 24 hours**

Compliance rate
98%

Average
5.9 mmol/L

CGM XXXX

Today's blood
glucose

Blood glucose
management

Me

FIG. 8B-1

User interface 820

FIG. 8B-2

| Health detection device | Terminal device |
|---|---|

S901: Obtain to-be-transmitted data, and divide the to-be-transmitted data into a first user health data set and a second user health data set based on detection time points

S902: Sample the first user health data set at a first data sampling interval, to obtain first transmission data

S903: First transmission data

S903: Output the first transmission data

S904: Fourth transmission data

S904: Output the fourth transmission data

S905: Sample the second user health data set at a second data sampling interval, to obtain second transmission data

S906: Second transmission data

S906: Output the second transmission data

S907: Third transmission data

S907: Output the third transmission data

FIG. 9

User interface 1010

FIG. 10A-1

User interface 1020

Today's blood glucose

5.2 mmol/L    02/09 08:07

Second time interval

Blood glucose

Time

**Last 24 hours**

Compliance rate
98%

Average
5.9 mmol/L

CGM XXXX

Today's blood
glucose

Blood glucose
management

Me

FIG. 10A-2

User interface 1010

**Today's blood glucose**

5.2 mmol/L  02/09 08:07

**Today's blood glucose**

Blood glucose

Time

| Last 24 hours | |
| --- | --- |
| Compliance rate 98% | Average 5.9 mmol/L |

CGM XXXX  >

Today's blood glucose

Blood glucose management

Me

FIG. 10B-1

User interface 1020

**Today's blood glucose**

5.2 mmol/L    02/09 08:07

**Second time interval**

Blood glucose

Time

**Last 24 hours**

Compliance rate
98%

Average
5.9 mmol/L

CGM XXXX

Today's blood
glucose

Blood glucose
management

Me

FIG. 10B-2

User interface 1010

FIG. 10C-1

User interface 1020

FIG. 10C-2

User interface 1010

**Today's blood glucose**

5.2 mmol/L   02/09 08:07

**Today's blood glucose**

Blood glucose

Time

| Last 24 hours | |
|---|---|
| Compliance rate 98% | Average 5.9 mmol/L |

CGM XXXX

Today's blood glucose | Blood glucose management | Me

FIG. 10D-1

User interface 1020

**Today's blood glucose**

5.2 mmol/L   02/09 08:07

**Second time interval**

Blood glucose

Time

**Last 24 hours**

| Compliance rate | Average |
|---|---|
| 98% | 5.9 mmol/L |

CGM XXXX

Today's blood glucose

Blood glucose management

Me

FIG. 10D-2

FIG. 11

Electronic device 100

Antenna 1

Antenna 2

FIG. 12

| Application layer | Camera | Calendar | Maps | WLAN | Music | Messages |
|---|---|---|---|---|---|---|
| | Gallery | Call | Navigation | Bluetooth | Videos | ... |

| Application frame layer | Window manager | Content provider | Phone manager | Resource manager |
|---|---|---|---|---|
| | Notification manager | View system | ... | |

| System library | Surface manager | Three-dimensional graphics processing library | Android runtime |
|---|---|---|---|
| | Two-dimensional graphics engine | Media library ... | |

| Kernel layer | Display driver | Camera driver | |
|---|---|---|---|
| | Audio driver | Sensor driver | ... |

FIG. 13

## INTERNATIONAL SEARCH REPORT

| International application No. |
|---|
| **PCT/CN2024/088027** |

**A. CLASSIFICATION OF SUBJECT MATTER**

G16H 40/67(2018.01)i

According to International Patent Classification (IPC) or to both national classification and IPC

**B. FIELDS SEARCHED**

Minimum documentation searched (classification system followed by classification symbols)

IPC:G16H

Documentation searched other than minimum documentation to the extent that such documents are included in the fields searched

Electronic data base consulted during the international search (name of data base and, where practicable, search terms used)

VEN; CNABS; CNTXT; WOTXT; EPTXT; USTXT; CNKI; IEEE: 数据, 传输, 用户, 健康, 检测, 检查, 时间点, 采样, 间隔, 数据量, 速度, 等待, 期望, 希望, 时长, 确定, 终端, 设备, user, data, transmission, health, detect, check, time, sampling, space, speed, wait, expectation, terminal, equipment

**C. DOCUMENTS CONSIDERED TO BE RELEVANT**

| Category* | Citation of document, with indication, where appropriate, of the relevant passages | Relevant to claim No. |
|---|---|---|
| A | CN 110911018 A (SHANDONG SPORT UNIVERSITY) 24 March 2020 (2020-03-24) description, paragraphs [0031]-[0087] | 1-24 |
| A | CN 114020186 A (HONOR TERMINAL CO., LTD.) 08 February 2022 (2022-02-08) entire document | 1-24 |
| A | CN 113496766 A (HUAWEI TECHNOLOGIES CO., LTD.) 12 October 2021 (2021-10-12) entire document | 1-24 |
| A | CN 113242712 A (HUAWEI TECHNOLOGIES CO., LTD.) 10 August 2021 (2021-08-10) entire document | 1-24 |
| A | WO 2022237598 A1 (HUAWEI TECHNOLOGIES CO., LTD.) 17 November 2022 (2022-11-17) entire document | 1-24 |

☐ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **19 June 2024** | **28 June 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

| International application No. |
| --- |
| **PCT/CN2024/088027** |

| Patent document cited in search report | | | Publication date (day/month/year) | Patent family member(s) | | | Publication date (day/month/year) |
| --- | --- | --- | --- | --- | --- | --- | --- |
| CN | 110911018 | A | 24 March 2020 | None | | | |
| CN | 114020186 | A | 08 February 2022 | None | | | |
| CN | 113496766 | A | 12 October 2021 | None | | | |
| CN | 113242712 | A | 10 August 2021 | WO | 2019101222 | A2 | 31 May 2019 |
| | | | | WO | 2019101222 | A3 | 08 August 2019 |
| WO | 2022237598 | A1 | 17 November 2022 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310461060